# EUROPEAN PATENT SPECIFICATION

(11) **EP 2 532 741 B1**
(45) Date of publication and mention of the grant of the patent: **04.04.2018**
(21) Application number: 11739572.3
(22) Date of filing: 03.02.2011
(51) Int. Cl.: C12N 5/074, A61K 35/407, C12N 5/071

(54) **INDUCED HEPATIC STEM CELL AND PROCESS FOR PRODUCTION THEREOF, AND APPLICATIONS OF THE CELL**
INDUZIERTE BLUTSTAMMZELLEN UND HERSTELLUNGSVERFAHREN DAFÜR SOWIE ANWENDUNGEN DIESER ZELLEN
CELLULE SOUCHE HÉPATIQUE INDUITE ET PROCÉDÉ POUR LA PRODUCTION DE CELLE-CI, ET APPLICATIONS DE LA CELLULE

(30) Priority: 03.02.2010 JP 2010022600
(43) Date of publication of application: 12.12.2012
(73) Proprietor: Ishikawa, Tetsuya, Suginami-ku Tokyo (JP)
(72) Inventor: ISHIKAWA, Tetsuya, Tokyo 104-0045 (JP); HAGIWARA, Keitaro, Tokyo 104-0045 (JP); OCHIYA, Takahiro, Tokyo 104-0045 (JP)
(74) Representative: Vossius & Partner Patentanwälte Rechtsanwälte mbB
(86) International application number: PCT/JP2011/000621
(87) International publication number: WO 2011/096223

(56) References cited:
- US-A1- 2004 191 902
- KARIM SI-TAYEB ET AL: "Highly efficient generation of human hepatocyte-like cells from induced pluripotent stem cells", HEPATOLOGY, WILEY, USA, vol. 51, no. 1, 1 January 2010 (2010-01-01), pages 297-305, XP002667016, ISSN: 0270-9139, DOI: 10.1002/HEP.23354 [retrieved on 2009-10-01]
- SONG Z ET AL: "Efficient generation of hepatocyte-like cells from human induced pluripotent stem cells", CELL RESEARCH CHINA, , vol. 19, no. 11 8 September 2009 (2009-09-08), pages 1233-1242, XP008155513, ISSN: 1748-7838, DOI: 10.1038/CR.2009.107 Retrieved from the Internet: URL:http://www.nature.com/cr/journal/v19/n 11/pdf/cr2009107a.pdf [retrieved on 2013-09-04]
- GARETH J SULLIVAN ET AL: "Generation of functional human hepatic endoderm from human induced pluripotent stem cells", HEPATOLOGY, WILEY, USA , vol. 51, no. 1 1 January 2010 (2010-01-01), pages 329-335, XP008161307, ISSN: 0270-9139, DOI: 10.1002/HEP.23335 Retrieved from the Internet: URL:http://onlinelibrary.wiley.com/doi/10. 1002/hep.23335/pdf [retrieved on 2009-09-29]
- CAI JUN ET AL: "Directed differentiation of human embryonic stem cells into functional hepatic cells", HEPATOLOGY, WILEY, USA, vol. 45, no. 5, 1 May 2007 (2007-05-01), pages 1229-1239, XP002454566, ISSN: 0270-9139, DOI: 10.1002/HEP.21582
- SI-TAYEB K ET AL.: 'Highly efficient generation of human hepatocyte-like cells from induced pluripotent stem cells.' HEPATOLOGY vol. 51, no. 1, January 2010, pages 297 - 305, XP002667016
- SULLIVAN G J ET AL.: 'Generation of functional human hepatic endoderm from human induced pluripotent stem cells.' HEPATOLOGY vol. 51, no. 1, January 2010, pages 329 - 335, XP008161307
- TAKAHIRO OCHIYA: 'Kan Saibo Bunka Shikosei no Takai Hito Hepa-iPS Saibo no Sakusei' DRUG DELIVERY SYSTEM vol. 25, no. 3, May 2010, page 237, XP008161309

## Description

### TECHNICAL FIELD

The present invention is defined by the claims. In more detail, the present invention relates to an induced hepatic stem cell as defined by the claims, a test using this induced hepatic stem cell as defined in the claims, and this induced hepatic stem cell for use in a therapeutic method of tertment of a mammal as defined in the claims. In general described herein are induced hepatic stem cells useful in safety tests, toxicity tests, metabolism tests, drug interaction tests, antiviral activity tests, screening tests for pharmaceuticals such as hyperlipidemic therapeutics, hypertension therapeutics, low-molecular weight compound medicaments, and antibody medicaments, screening for targets in drug discovery, preparation of animal models, production of hepatocyte-produced proteins, and in regenerative medicine; in particular, described are induced hepatic stem cells that have properties of hepatocytes, that express a group of marker genes for embryonic stem cells in comparable amounts to embryonic stem cells, and which can be subjected to expansion culture and passage culture over a prolonged period; also described herein are processes for producing such induced hepatic stem cells and applications of such cells.

### BACKGROUND ART

Research and development of new drugs by pharmaceutical companies suffer not only from prolongation of the R&D period but also from the increasing R&D costs. What is more, despite a number of candidates that are expected to be new drugs in the future, problems will arise in terms of effectiveness and safety as the R&D activities proceed and the development of most candidates has to be given up.

The development of new drugs generally requires long term ranging from 9 to 17 years and huge amounts of R&D costs on the order of a billion to ten billions, so a drug discovery tool capable of narrowing down candidate compounds at an early stage such as a preclinical stage would lead to a lower development cost.

However, in current non-clinical tests, it is necessary to perform animal tests to evaluate the safety, toxicity and other features of the drug under test and this is one factor that leads to the soaring development cost. What is more, the *in vivo* kinetics of the drug might differ on account of the species differences between human and other animals, making it difficult to perform sufficient evaluation of safety, so it is only in the clinical test stage that the candidate compound is found to have toxicity, and this again causes the development effort to be given up.

While there is a strong need to establish a system by which *in vivo* kinetics and the like of a candidate compound in humans can be predicted and evaluated at an early stage of the research and development processes, efforts are now being made in order to construct an evaluation system that uses human hepatocytes to replace animal experiments which are limited by the "barrier of species differences." By using this evaluation system, candidate compounds for the drugs under development can be accurately limited to highly safe candidate drugs at an early stage of the development, so pharmaceutical companies have a particularly great demand for the system.

In conventional non-clinical tests using human cultured cells, primary cultured hepatocytes or existing cell lines from non-Japanese people have been employed. However, primary cultured hepatocytes have the problems of an overwhelming scarcity of donors and exceedingly great lot differences. In particular, primary cultured hepatocytes from Japanese people, which involve ethical issues and are regulated by law, are extremely difficult to obtain and their consistent supply is impossible.

Furthermore, drug metabolizing enzymes that are expressed in the liver tissue play an important role in catalyzing the metabolism of many pharmaceuticals. However, on account of the accompanying polymorphisms are present and the amount of their expression and their activity are affected by significant individual differences, these factors make the above-mentioned problem with non-clinical tests even more serious.

To cope with the differences present among a huge number of individuals, it is desirable that primary cultured hepatocytes derived from a plurality of donors who cover such differences can be repeatedly used as representative cells in various types of tests. However, primary cultured hepatocytes can hardly be expanded on a culture dish and this presents a problem in that it is practically difficult to perform passage culture of the same hepatocyte and use it repeatedly in various tests.

In contrast, many of the existing established cell lines are those cells which have experienced karyotypic abnormality and there are not many enough cell lines to cover the differences among a huge number of individuals. Moreover, the existing established cell lines subjected to prolonged passage culture by conventional methods do not show the same drug metabolizing enzyme activity or transporter inducing ability as the primary culturd hepatocytes, so given this result, it is impossible to predict the safety, metabolism, and other features in humans in clinical applications.

Under these circumstances, cells are desired that have properties of hepatocytes and which can be subjected to passage culture for a prolonged period. However, there have ever been no report to show that such cells were discovered from a plurality of donors who cover the differences among a huge number of individuals.

As regards stem cells for the liver, the existence of hepatic stem cells having an ability to differentiate into to hepatocytes is assumed. Again, however, there has ever been no report to show that hepatic stem cells were discovered that have such a nature that they express self-replicating genes like embryonic stem cells and induced pluripotent stem cells and can be subjected to passage culture *ex vivo* for a prolonged period.

Hence, research is being conducted to determine whether embryonic stem cells (ES cells) that retain the pluripotency to differentiate into somatic cells of theoretically all tissues and gem cells and which yet are capable of self-replication almost unlimitedly in an undifferentiated state might be applicable not only in regenerative medicine but also in the field of drug discovery. Embryonic stem cells refers to a stem cell line prepared from inner cell masses belonging to part of the embryo in a blastocyst stage which is an early development stage of an animal and they are sometimes called by the acronym ES cells.

To establish embryonic stem cells, a fertilized egg or an early embryo at any of the stages up to a blastocyst which is more developed than the fertilized egg is required. In the case of humans, a fertilized egg is used as the starting material and the resulting loss of emerging potential of human life is recognized to pose an ethical issue. For this reason, some countries prohibit making any study, including preparation, of human embryonic stem cells; even in countries that permit research on human embryonic stem cells with the recognition of their potential to treat neurodegenerative diseases (e.g., Parkinson's disease) and spinal cord injury, i.e., the diseases the radical therapy for which has not yet been established, strict limitations are put on handling human embryonic stem cells. Thus, ethical issues constitute high barriers to both foundamental and applied research on embryonic stem cells.

In addition, embryonic stem cells need be differentiated into certain specific cells before they can be put to practical application, and methods for differentiating them into hepatocytes, nerve cells, cardiomyocytes, pancreatic beta cells, and the like are being increasingly developed. However, differentiation into these specific cells is difficult to realize and induced differentiation to hepatocytes is particularly difficult. As of the present, no method has been established that enables highly efficient induction of differentiation to mature hepatic stem cells that has sufficiently high quality to be used in drug discovery research. All methods that have been so far reported to be capable of inducing differentiation require as many as about three weeks to induce differentiation. However, the hepatocyte-like cells that have been highly induced differentiation with taking extensive cost, much labor, and lengthy time can hardly be increased in number.

It has recently been reported that by introducing OCT3/4 gene (OCT3/4 is a gene's name and sometimes designated as OCT3 or OCT4 but it is hereinafter referred to as POUF1 gene), SOX2 gene, KLF4 gene, and c-MYC gene (Patent Document 1) or by introducing POU5F1 gene, SOX2 gene, and KLF4 gene in the presence of a basic fibroblast growth factor (Non-Patent Document 1), induced pluripotent stem cells which are undifferentiated cells as embryonic stem cells can be prepared from somatic cells in human and the like (Patent Document 2). Human induced pluripotent stem cells (iPS cells) are known to have two characteristic features, (1) pluripotency for differentiation into three germ layers (i.e., endoderm. mesoderm, and ectoderm) which are capable of becoming all cells that form a body and (2) self-replicating ability by which the cells can be subjected to passage culture unlimitedly in a culture dish under specified conditions while remaining undifferentiated state. It also has been reported that such human induced pluripotent stem cells are very similar to human embryonic stem cells in terms of morphology, gene expression, cell surface antigen, long-term self-replicating ability, and teratoma (benign tumor) forming ability (Non-Patent Documents 2 and 3), as well as that the genotypes of HLA are identical to those of somatic cells which are derived cells (Non-Patent Document 3).

In the preparation of such induced pluripotent stem cells, it is held that a differentiated somatic cell can be "reset or reprogrammed" to an undifferentiated pluripotent stem cell by simply introducing four genes, (i.e., POUF1 gene, SOX2 gene, KLF4 gene, and c-MYC gene) or three genes (i.e., POU5F1 gene, SOX2 gene, and KLF4 gene) into the cell. However, in human cells, induced pluripotent stem cells are prepared from somatic cells at an efficiency of 0.1%-0.01% in the case of four-gene transfection, and at a 0.01%-0.001% efficiency in three-gene transfection. This means that 99.9%-99.999% cells will not be reprogrammed to an induced pluripotent stem cell by gene transfer.

Moreover, NANOG gene, POU5F1 gene, SOX2 gene, ZFP42 gene, SALL4 gene, LIN28 gene, and TERT gene, and the like that are characteristically expressed in embryonic stem cells have been held to be important factors for pluripotent stem cells to remain undifferentiated state, thus serving to suppress cell differentiation. Therefore, in a differentiated cell, the expression of NANOG gene, POUF1 gene, SOX2 gene, ZFP42 gene, SALL4 gene, LIN28 gene, and TERT gene which are held to be important factors in the maintenance of an undifferentiated state will disappear as differentiation genes (properties of the differentiated cell) are expressed.

In other words, it has been considered to be impossible to prepare a cell in which not only NANOG gene, POUF1 gene, SOX2 gene, ZFP42 gene, SALL4 gene, LIN28 gene, and TERT gene which are held to be important factors in the maintenance of a undifferentiated state, but also many properties of a differentiated cell (many differentiation genes) are expressed.

### CITATION LIST

### PATENT DOCUMENTS

Patent Document 1: JP 2008-283972 A
Patent Document 2: JP 2008-307007

### NON-PATENT DOCUMENTS

Non-Patent Document 1: Nakagawa M et al., Nat Biotechnol., 2008, 26,101-6
Non-Patent Document 2: Takahashi K, Yamanaka S et al., Cell, 2007, 131, 861-872
Non-Patent Document 3: Masaki H, Ishikawa T et al., Stem Cell Res., 2008, 1, 105-115

Under these circumstances, the present inventors carried out an intensive study to know whether it was possible to prepare cells having both the genes important for the maintenance of an undifferentiated state and many properties of hepatocytes; as a result, they found that it was possible to prepare induced hepatic stem cells that expressed genes characteristic of embryonic stem cells and which yet expressed genes characteristic of hepatocytes; in addition, they found that these induced hepatic stem cells were useful in safety tests, toxicity tests, metabolism tests, drug interaction tests, antiviral activity tests, screening tests for pharmaceuticals such as hyperlipidemic therapeutics, hypertension therapeutics, low-molecular weight compound medicaments, and antibody medicaments, screening for targets in drug discovery, preparation of animal models, production of hepatocyte-produced proteins, and in regenerative medicine; the present invention has been accomplished accordingly.

### SUMMARY OF INVENTION

### TECHNICAL PROBLEMS

Therefore, a first object of the present invention is to provide an induced hepatic stem cell that expresses genes characteristic of an embryonic stem cell and which yet expresses genes characteristic of a hepatocyte.

A second object of the present invention is to provide a process for preparing an induced hepatic stem cell that expresses genes characteristic of an embryonic stem cell and which yet expresses genes characteristic of a hepatocyte.

A third object of the present invention is to provide methods using the induced hepatic stem cell of the present invention, including safety test methods, toxicity test methods, metabolism test methods, drug interaction test methods, antiviral activity test methods, screening test methods for pharmaceuticals such as hyperlipidemic therapeutics, hypertension therapeutics, low-molecular weight compound medicaments, and antibody medicaments, methods of screening for targets in drug discovery, methods for preparation of animal models, methods for production of hepatocyte-produced proteins, and methods of regenerative medicine.

### SOLUTION TO PROBLEMS

Thus, a first aspect of the present invention relates to an induced hepatic stem cell obtained by a process comprising a step of inducing a mammalian cell into an induced hepatic stem cell, wherein the mammalian cell is selected from the group consisting of an adult-derived cell, a neonate-derived cell, skin-derived cell, and a cell from a cancerous individual, wherein said step of inducing comprises introducing POU5F1 gene, KLF4 gene, and SOX2 gene into the mammalian cell with the aid of expression vectors to bring the mammalian cell to such a state that gene products of the POU5F1 gene, the KLF4 gene, and the SOX2 gene which are necessary for induction to the induced hepatic stem cell will be present to ensure that the intracellular relative abundance of the gene product of POU5F1 gene is greater than that of the gene product of SOX2 gene, wherein the ratio in use between POUF1 gene, KLF4 gene, and SOX2 gene which are necessary for induction to the induced hepatic stem cell is 4:2:1 in that order, and wherein the induced hepatic stem cell is characterized by at least satisfying the following requirements (1)-(3):
(1) it expresses at least 15 genes as selected from the group of the genes listed in the following Table 1 which are marker genes for an embryonic stem cell;

**[Table 1]**

| GeneSymbol | GenbankAccession |
|---|---|
| ACVR2B | NM_001106 |
| CD24 | L33930 |
| CDH1 | NM_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | MM_020634 |
| GRB7 | NM_005310 |
| UN28 | NM_024674 |
| NANOG | NM_024865 |
| NODAL | NM_018055 |
| PODXL | NM_005397 |
| POU5F1 | NM_002701 |
| SALL4 | NM_020436 |
| SOX2 | NM_003106 |
| TDGF1 | NM_003212 |
| TERT | NM_198253 |
| ZFP42 | NM_174900 |
| ZIC3 | NM_003413 |

(2) it has properties of a hepatocyte wherein at least 15 genes as selected from the gene group in Table 2 below are expressed as genes associated with the properties of a hepatocyte in (2) above.

**[Table 2]**

| GeneSymbol | GenbankAccession | GeneSymbol | GenbankAccession | GeneSymbol | GenbankAccession |
|---|---|---|---|---|---|
| A2M | NM_000014 | ERP27 | NM_152321 | NRCAM | NM_005010 |
| ACE2 | NM_021804 | EVA1 | NM_144765 | NTF3 | NM_002527 |
| ACVRL1 | NM_000020 | F10 | NM_000504 | OLFML2A | NM_182487 |
| ADAMTS9 | NM_182920 | F2 | NM_000506 | PAG1 | NM_018440 |
| AFAP1L2 | NM_001001936 | FABP1 | NM_001443 | PCSK6 | NM_002570 |
| AFP | NM_001134 | FGA | NM_021871 | PDK4 | NM_002612 |
| AGT | NM_000029 | FGA | NM_000508 | PDZK1 | NM_002614 |
| AHSG | NM_001622 | FGB | NM_005141 | PLA2G12B | NM_032562 |
| AK027294 | AK027294 | FGG | NM_000509 | PLG | NM_000301 |
| AK074614 | AK074614 | FLRT3 | NM_198391 | PRG4 | NM_005807 |
| AK124281 | AK124281 | FMOD | NM_002023 | PSMAL | NM_153696 |
| AK126405 | AK126405 | FOXA1 | NM_004496 | PTGDS | NM_000954 |
| ALB | NM_000477 | FTCD | NM_206965 | PTHR1 | NM_000316 |
| ALDH1A1 | NM_000689 | GATA4 | NM_002052 | RASD1 | NM_016084 |
| ANXA8 | NM_001630 | GATM | NM_001482 | RBP4 | NM_006744 |
| APCDD1 | NM_153000 | GDF10 | NM_004962 | RNF43 | NM_017763 |
| APOA1 | NM_000039 | GJB1 | NM_000166 | RRAD | NM_004165 |
| APOA2 | NM_001643 | GLT1D1 | NM_144669 | S100A14 | NM_020672 |
| APOA4 | NM_000482 | GPRC5C | NM_022036 | SEPP1 | NM_005410 |
| APOB | NM_000384 | GSTA3 | NM_000847 | SERINC2 | NM_178865 |
| AREG | NM_001657 | GUCY1A3 | NM_000856 | SERPINA1 | NM_001002236 |
| ART4 | NM_021071 | H19 | NR_002196 | SERPINA3 | |
| ASGR2 | NM_080912 | HHEX | NM_002729 | SERPINA5 | |
| ATAD4 | NM_024320 | HKDC1 | NM_025130 | SH3TC1 | NM_018986 |
| BC018589 | BC018589 | HMGCS2 | NM_005518 | SLC13A5 | NM_177550 |
| BMP2 | NM_001200 | HP | NM_005143 | SLC40A1 | NM_014585 |
| BX097190 | BX097190 | HPR | NM_020995 | SLC5A9 | |
| C11orf9 | NM_013279 | HPX | NM_000613 | SLCO2B1 | MM_007256 |
| C13orf15 | NM_014059 | HSD17B2 | NM_002153 | SLP1 | NM_003064 |
| C15orf27 | NM_152335 | HTRA3 | NM_053044 | SPARCL1 | |
| C3 | NM_000064 | IGF2 | NM_001007139 | SPON1 | NM_006108 |
| C5 | NM_001735 | IL32 | NM_001012631 | ST8SIA1 | NM_003034 |
| CA414006 | CA414006 | INHBB | NM_002193 | STARD10 | NM_006645 |
| CD163 | NM_004244 | ISX | NM_001008494 | STMN2 | S82024 |
| CD1D | NM_001766 | KCNJ16 | NM_170741 | TD02 | NM_005651 |
| CDX2 | NM_001265 | KYNU | NM_003937 | TF | NM_001063 |
| CILP | NM_003613 | LAMC2 | NM_005562 | TMC6 | NM_007267 |
| CMKLR1 | NM_004072 | LGALS2 | NM_006498 | TMEM16D | NM_178826 |
| COL4A6 | NM_033841 | LHX2 | NM_004789 | TSPAN15 | NM_012339 |
| COLEC11 | NM_199235 | LOC132205 | AK091178 | TTR | NM_000371 |
| CXCL14 | NM_004887 | LOC285733 | AK091900 | UBD | NM_006398 |
| CXCR4 | NM_001008540 | M27126 | M27126 | UGT2B11 | NM_001073 |
| CXCR7 | NM_020311 | MAF | AF055376 | UGT2B7 | NM_001074 |
| DACH1 | NM_080759 | MFAP4 | NM_002404 | UNC93A | NM_018974 |
| DENND2A | NM_015689 | MMP10 | NM_002425 | VCAM1 | NM_001078 |
| DIO3 | NM_001362 | MTTP | NM_000253 | VIL1 | NM_007127 |
| DLK1 | NM_003836 | NGEF | NM_019850 | VTN | |
| DUSP6 | NM_001946 | NGFR | NM_002507 | WFDC1 | |

(3) it can be subjected to expansion culture or passage culture for at least 3 days.

Preferably the marker genes for an embryonic stem cell in (1) above are expressed in the induced hepatic stem cell in amounts ranging from 1/8-8 times the amounts of the genes that are expressed in the embryonic stem cell (claim 2). More preferably the marker genes for an embryonic stem cell in (1) above are expressed in the induced hepatic stem cell in amounts ranging from 1/4-4 times the amounts of the genes that are expressed in the embryonic stem cell (claim 3).

**[Table 2]**

| GeneSymbol | GenbankAccession | GeneSymbol | GenbankAccession | GeneSymbol | GenbankAccession |
|---|---|---|---|---|---|
| A2M | NM_000014 | ERP27 | NM_152321 | NRCAM | NM_005010 |
| ACE2 | NM_021804 | EVA1 | NM_144765 | NTF3 | NM_002527 |
| ACVRL1 | NM_000020 | F10 | NM_000504 | OLFML2A | NM_182487 |
| ADAMTS9 | NM_182920 | F2 | MM_000506 | PAG1 | NM_018440 |
| AFAP1L2 | NM_001001936 | FABP1 | NM_001443 | PCSK6 | NM_002570 |
| AFP | NM_001134 | FGA | NM_021871 | PDK4 | NM_002612 |
| AGT | NM_000029 | FGA | NM_000508 | PDZK1 | NM_002614 |
| AHSG | NM_001622 | FGB | NM_005141 | PLA2G12B | NM_032562 |
| AK027294 | AK027294 | FGG | NM_000509 | PLG | NM_000301 |
| AK074614 | AK074614 | FLRT3 | NM_198391 | PRG4 | NM_005807 |
| AK124281 | AK124281 | FMOD | NM_002023 | PSMAL | NM_153696 |
| AK126405 | AK126405 | FOXA1 | NM_004496 | PTGDS | NM_000954 |
| ALB | NM_000477 | FTCD | NM_206965 | PTHR1 | NM_000316 |
| ALDH1A1 | NM_000689 | GATA4 | NM_002052 | RASD1 | NM_016084 |
| ANXA8 | NM_001630 | GATM | NM_001482 | RBP4 | NM_006744 |
| APCDD1 | NM_153000 | GDF10 | NM_004962 | RNF43 | NM_017763 |
| APOA1 | NM_000039 | GJB1 | NM_000186 | RRAD | NM_004165 |
| APOA2 | NM_001643 | GLT1D1 | NM_144669 | S100A14 | NM_020672 |
| APOA4 | NM_000482 | GPRC5C | NM_022036 | SEPP1 | NM_005410 |
| APOB | NM_000384 | GSTA3 | NM_000847 | SERINC2 | NM_178865 |
| AREG | NM_001657 | GUCY1A3 | NM_000856 | SERPINA1 | NM_001002236 |
| ART4 | NM_021071 | H19 | NR_002196 | SERPINA3 | NM_001085 |
| ASGR2 | NM_080912 | HHEX | NM_002729 | SERPINA5 | NM_000624 |
| ATAD4 | NM_024320 | HKDC1 | NM_025130 | SH3TC1 | NM_018986 |
| BC018589 | BC018589 | HMGCS2 | NM_005518 | SLC13A5 | NM_177550 |
| BMP2 | NM_001200 | HP | NM_005143 | SLC40A1 | NM_014585 |
| SX097190 | BX097190 | HPR | NM_020995 | SLC5A9 | NM_001011547 |
| C11orf9 | NM_013279 | HPX | NM_000813 | SLCO2B1 | NM_007256 |
| C13orf15 | NM_014059 | HSD17B2 | NM_002153 | SLPI | NM_003064 |
| C15orf27 | NM_152335 | HTRA3 | NM_053044 | SPARCL1 | NM_004684 |
| C3 | NM_000064 | IGF2 | NM_001007139 | SPON1 | NM_006108 |
| C5 | NM_001735 | IL32 | NM_001012631 | ST8SIA1 | NM_003034 |
| CA414006 | CA414006 | INHBB | NM_002193 | STARD10 | NM_006645 |
| CD163 | NM_004244 | ISX | NM_001008494 | STMN2 | S82024 |
| CD1D | NM_001766 | KCNJ16 | NM_170741 | TD02 | NM_005651 |
| CDX2 | NM_001265 | KYNU | NM_003937 | TF | NM_001063 |
| CILP | NM_003613 | LAMC2 | NM_005562 | TMC6 | NM_007267 |
| CMKLR1 | NM_004072 | LGALS2 | NM_006498 | TMEM16D | NM_178826 |
| COL4A6 | NM_033641 | LHX2 | NM_004789 | TSPAN15 | NM_012339 |
| COLEC11 | NM_199235 | LOC132205 | AK091178 | TTR | NM_000371 |
| CXCL14 | NM_004887 | LOC285733 | AK091900 | UBD | NM_006398 |
| CXCR4 | NM_001008540 | M27126 | M27126 | UGT2B11 | NM_001073 |
| CXCR7 | NM_020311 | MAF | AF055376 | UGT2B7 | NM_001074 |
| DACH1 | NM_080759 | MFAP4 | NM_002404 | UNC93A | NM_018974 |
| DENND2A | NM_015689 | MMP10 | NM_002425 | VCAM1 | NM_001078 |
| DIO3 | NM_001362 | MTTP | NM_000253 | VIL1 | NM_007127 |
| DLK1 | NM_003836 | NGEF | NM_019850 | VTN | NM_000638 |
| DUSP6 | NM_001946 | NGFR | NM_002507 | WFDC1 | NM_021197 |

NANOG gene, POUF1 gene, SOX2 gene, ZFP42 gene, and SALL4 gene are preferably expressed as the marker genes for an embryonic stem cell in (1) above (claim 4). AFP gene, TTR gene, TF gene, APOA2 gene, APOA4 gene, AHSG gene, FGA gene, AGT gene, FABP1 gene, SERPINA1 gene, and RBP4 gene are preferably expressed as genes associated with the properties of a hepatocyte in (2) above (claim 5). In another embodiment APOA1 gene, APOA2 gene, APOA4 gene, APOB gene, FABP1 gene, AGT gene are preferably expressed as genes associated with the properties of a hepatocyte in (2) above (claim 6).

Preferably, the induced hepatic stem cell of the present invention further expresses at least one gene as selected from among SOX17 gene, FOXA2 gene, GSC gene, EOMES gene, and TCF2 gene which are characteristic of mesendodermal stem cells and/or endodermal stem cells (claim 7). It also described herein that at least one gene as selected from the gene group in Table 3 below has its expression suppressed or induced, or has the activity of a gene product of said gene promoted or inhibited, by a test substance.

**[Table 3]**

| GeneSymbol | GenbankAccession | GeneSymbol | GenbankAccession | GeneSymbol | GenbankAccession |
|---|---|---|---|---|---|
| ABCB1 | NM_000927 | GSTA1 | NM_145740 | SLC22A6 | NM_153277 |
| ABCB11 | NM_003742 | GSTA2 | NM_000846 | SLC22A7 | NM_153320 |
| ABCB4 | NM_018850 | GSTA3 | NM_000847 | SLC22A8 | NM_004254 |
| ABCC1 | NM_019862 | GSTA4 | NM_001512 | SLC22A9 | NM_080866 |
| ABCC2 | NM_000392 | GSTA5 | NM_153699 | SLCO1A2 | NM_005075 |
| ABCC3 | NM_003786 | GSTM1 | NM_146421 | SLCO1A2 | NM_134431 |
| ACTB | NM_001101 | GSTM2 | NM_000848 | SLCO1B1 | NM_008446 |
| AHR | NM_001621 | GSTM3 | NM_000849 | SLCO1B3 | NM_019844 |
| ARNT | NM_001688 | GSTM4 | NM_147148 | SLCO1C1 | NM_017435 |
| BAAT | NM_001701 | GSTM5 | NM_000851 | SLCO2A1 | NM_005630 |
| COMT | NM_000754 | GSTP1 | NM_000852 | SLCO2B1 | NM_007256 |
| CYP1A1 | NM_000499 | GSTT1 | NM_000853 | SLCO3A1 | XM_001132480 |
| CYP1A2 | NM_000761 | GSTT2 | NM_000854 | SLCO3A1 | NM_013272 |
| CYP1B1 | NM_000104 | GSTZ1 | NM_145870 | SLCO4A1 | NM_016354 |
| CYP2A13 | NM_000766 | NAT1 | NM_000662 | SLCO4C1 | NM_180991 |
| CYP2A6 | NM_000762 | NAT2 | NM_000015 | SULT1A1 | NM_177529 |
| CYP2A7 | NM_000764 | NR1H4 | NM_005123 | SULT1A2 | NM_177528 |
| CYP2B6 | NM_000767 | NR1I2 | NM_003889 | SULT1A3 | AK094769 |
| CYP2C18 | NM_000772 | NR1I3 | NM_005122 | SULT1A4 | NM_001017389 |
| CYP2C19 | NM_000769 | PPARA | NM_005036 | SULT1B1 | D89479 |
| CYP2C8 | NM_000770 | PPARA | L02932 | SULT1B1 | NM_014465 |
| CYP2C9 | NM_000171 | PPARD | NM_006238 | SULT1C2 | NM_176825 |
| CYP2D6 | NM_000106 | PPARG | NM_138711 | SULT1C4 | NM_006588 |
| CYP2E1 | NM_000773 | RPL13 | NM_033251 | SULT1E1 | NM_005420 |
| CYP2F1 | NM_000774 | RPS18 | NM_022551 | SULT2A1 | NM_003167 |
| CYP2J2 | NM_000775 | RXRA | NM_002957 | SULT2B1 | NM_004605 |
| CYP3A4 | NM_017460 | RXRB | NM_021976 | SULT4A1 | NM_014351 |
| CYP3A5 | NM_000777 | RXRG | NM_008917 | TPMT | NM_000367 |
| CYP3A5 | AF355801 | SLC10A1 | NM_003049 | UGT1A6 | NM_001072 |
| CYP3A7 | NM_000765 | SLC10A2 | NM_000452 | UGT1A8 | NM_019076 |
| CYP4A11 | NM_000778 | SLC16A1 | NM_003051 | UGT2A1 | NM_006798 |
| CYP4B1 | NM_000779 | SLC17A1 | NM_005074 | UGT2B10 | NM_001075 |
| CYP4F11 | NM_021187 | SLC22A1 | NM_153187 | UGT2B11 | NM_001073 |
| CYP4F12 | NM_023944 | SLC22A10 | NM_001039752 | AGT2815 | NM_001076 |
| CYP4F2 | NM_001082 | SLC22A11 | AK075127 | UGT2B17 | NM_001077 |
| CYP4F3 | AB002454 | SLC22A11 | NM_018484 | UGT2B28 | NM_053039 |
| CYP4F8 | NM_007253 | SLC22A2 | NM_003058 | UGT2B4 | NM_021139 |
| EEF1A1 | NM_001402 | SLC22A3 | NM_021977 | UGT2B7 | NM_001074 |
| ENDOG | NM_004435 | SLC22A4 | NM_003059 | | |
| GAPDH | NM_002046 | SLC22A5 | NM_003060 | | |

It is also described herein that the induced hepatic stem cell described herein can be subjected to expansion culture or passage culture for at least a month.

Also described herein is a process for producing an induced hepatic stem cell comprising a step of inducing a mammalian cell to an induced hepatic stem cell, the step bringing the mammalian cell to such a state that gene products of POU5F1 gene, KLF4 gene, and SOX2 gene which are necessary for induction to the induced hepatic stem cell will be present to ensure that the intracellular relative abundance of the gene product of POU5F1 gene is greater than that of the gene product of SOX2 gene. The step may be such that it uses POUF1 gene, KLF4 gene, and SOX2 gene which are necessary for induction to the induced hepatic stem cell or gene products of these genes, and that the ratio in use of POUF1 gene or the gene product of this gene to SOX2 gene or the gene product of this gene is greater than one.

In accordance with the present invention the ratio in use between POUF1 gene, KLF4 gene, and SOX2 gene which are necessary for induction to the induced hepatic stem cell satisfies the relation of POU5F1 gene > KLF4 gene > SOX2 gene, more particularly with the ratio of 4:2:1 in that order (claim 1).

The foregoing mammalian cells to be used in the present invention is an adult-derived cell, a neonate-derived cell, a skin-derived cell, or a cancerous individual's cell (claim 1), with the mammal being preferably a human (claim 8).

A third aspect of the present invention relates to a test method using the induced hepatic stem cell of the present invention and the test method is a safety test method, a toxicity test method, a metabolism test method, a drug interaction test method, an antiviral activity test method, or a screening test method for pharmaceuticals such as hyperlipidemic therapeutics, hypertension therapeutics, low-molecular weight compound medicaments, and antibody medicaments (claim 9). Also described herein are a method of screening for targets in drug discovery, a method for preparation of an animal model, and a method for production of a hepatocyte produced protein. A seventh aspect relates to a therapeutic use directed to a mammal (claim 10).

### ADVANTAGEOUS EFFECT OF INVENTION

According to the present invention, human induced hepatic stem cells can be prepared from donors of different races, sexes, ages or genetic backgrounds, so the present invention is effective in non-clinical tests on new drugs, such as a safety test, a toxicity test, a metabolism test, and a drug interaction test, that are performed prior to clinical tests. In addition, non-clinical tests using the human induced hepatic stem cells provide drug discovery tools that contribute to more efficient development of new drugs.

### DESCRIPTION

On the pages that follow, the induced hepatic stem cell described herein, the process for its production, and the applications of that cell are described in detail.

The induced hepatic stem cell being described herein is characterized by satisfying at least the following three requirements (1) to (3):
(1) it expresses at least 15 genes as selected from the group of the genes listed in the foregoing Table 1 which are marker genes for an embryonic stem cell;
(2) it has properties of a hepatocyte;
(3) it can be subjected to expansion culture or passage culture for at least 3 days.

Next, in the induced hepatic stem cell described herein, the expression of the marker genes for an embryonic stem cell in (1) above which are known as marker genes for an embryonic stem cell serves to specify that the induced hepatic stem cell described herein is a cell having such a nature that it theoretically self-replicates unlimitedly and that it can be subjected to prolonged passage culture while substantially remaining as an induced hepatic stem cell. It is necessary that at least 15 genes as selected from the group of the genes listed in the foregoing Table 1 are necessarily to be expressed in the induced hepatic stem cell described herein.

The induced hepatic stem cell described herein is not particularly limited as long as the marker genes for an embryonic stem cell in (1) above are expressed in it, but the marker genes for an embryonic stem cell in (1) above may be expressed in the induced hepatic stem cell described herein in amounts ranging from 1/16-16 times the amounts of the genes that are expressed in the embryonic stem cell, with the range from 1/8-8 times being preferred. For the purpose of maintaining the state of the induced hepatic stem cell or from the viewpoint of prolonged passage culture, that the marker genes for an embryonic stem cell in (1) above may be expressed in the induced hepatic stem cell described herein in almost comparable amounts, namely amounts ranging from 1/4-4 times the amounts of the genes that are expressed in the embryonic stem cell, with the range from 1/2-2 being preferred.

The induced hepatic stem cell described herein is such that, from the viewpoint of maintaining an undifferentiated state, at least 15 genes selected from the group of the genes listed in the foregoing Table 1 as the marker genes for an embryonic stem cell in (1) above are expressed in the induced hepatic stem cell in amounts within the range from 1/2-2 the amounts of the genes that are expressed in the embryonic stem cell, and as the number of the marker genes for an embryonic stem cell in (1) above that are expressed within this range increases to 20, 25 or even more, the result becomes the better.

The induced hepatic stem cell described herein is such that, of the genes listed in the foregoing Table 1 as the marker genes for an embryonic stem cell in (1) above, five and more, or ten and more, or even twenty and more are preferably expressed in the induced hepatic stem cell in amounts within the range from 1/2-2 , from 1/4-4 times, and from 1/8-8 times, respectively, the amounts of the genes that are expressed in the embryonic stem cell.

The induced hepatic stem cell described herein is such that, among the genes listed in the foregoing Table 1 as the marker genes for an embryonic stem cell in (1) above, five genes including NANOG gene, POUF1 gene, and SOX2 may be expressed in the induced hepatic stem cell in amounts within the range from 1/4-4. times the amounts of the genes that are expressed in the embryonic stem cell; preferably, five genes (i.e., NANOG gene, POU5F1 gene, SOX2 gene, ZFP42 gene, and SALL4 gene) are expressed in amounts within the range from 1/4-4 times the amounts of the genes that are expressed in the embryonic stem cell; more preferably, ten genes (i.e., NANOG gene, POU5F1 gene, SOX2 gene, TDGF1 gene, DNMT3B gene, ZFP42 gene, TERT gene, GDF3 gene, SALL4 gene, and GABRB3 gene) are expressed in amounts within the range from 1/4-4 times the amounts of the genes that are expressed in the embryonic stem cell.

The aforementioned embryonic stem cell to be used as a reference for comparison is any one of hES_H9 (GSM194390), hES_BG03 (GSM194391), and hES_ES01 (GSM194392). Relevant data for gene expression can be accessed from the database Gene Expression Omnibus [GEO] ("Gene Expression Omnibus [GEO], [online], [searched on January 28, 2010], the internet <http://www.ncbi.nlm.nih.gov/geo/>).

The induced hepatic stem cell described herein is required to have properties of a hepatocyte in (2) above. Properties of a hepatocyte in the induced hepatic stem cell described herein are not particularly limited as long as they are properties characteristic of the hepatocyte, but a typical example is the production of proteins (gene products) that are characteristic of hepatocytes. Specific examples include, but are not limited to, the production of serum proteins (e.g., AFP, TTR, TF, APOA2, APOA4, AHSG, FGA, AGT, FABP1, SERPINA1, and RBP4), the production of enzymes associated with saccharide metabolism, amino acid metabolism, lipid metabolism, and iron metabolism, as well as the production of drug metabolizing enzymes and transporters.

The induced hepatic stem cell described herein may expresses genes in (2) above associated with the properties of a hepatocyte. These genes may be ones that are characteristically expressed in hepatocytes and which are associated with properties of the hepatocyte; they may be exemplified by genes that are associated with, for example, the production of proteins characteristic of hepatocytes. Specific examples include, but are not limited to, genes associated with the production of serum proteins, the production of enzymes associated with saccharide metabolism, amino acid metabolism, lipid metabolism, and iron metabolism, as well as the production of drug metabolizing enzymes and transporters, etc. In particular, the genes associated with the production of drug metabolizing enzymes and transporters include, for example, the group of genes listed in Table 3 above. Such genes associated with the production of drug metabolizing enzymes and transporters display gene expression, induction, suppression and the like in response to test substances such as candidate compounds for pharmaceuticals that have been taken up by the induced hepatic stem cell described herein.

In one example of the induced hepatic stem cell described herein, at least 15 genes that are liver-associated genes as selected from the group of genes in Table 2 above may be expressed as genes in (2) above associated with the properties of a hepatocyte. These genes are ones that are characteristic of hepatocytes and which are expressed in a human primary culture of hepatocytes.

GenBank accession numbers corresponding to the respective gene symbols are as listed in Table 2 above. Relevant gene information can be accessed from the web site of NCBI (http://www.ncbi.nlm.nih.gov/nucleotide/).

As regards the genes in (2) above associated with the properties of a hepatocyte, 50 or more of the genes listed in Table 2 above may be expressed from the viewpoint of the possibility of providing cells that strongly exhibit properties characteristic of hepatocytes, and it is preferred that 80 or more of such genes are expressed. In the induced hepatic stem cell described herein, AFP gene may be expressed among the genes listed in Table 2 above, and it is preferred that AFP gene, TTR gene, TF gene, APOA2 gene, APOA4 gene, AHSG gene, FGA gene, AGT gene, FABP1 gene, SERPINA1 gene, and RBP4 gene are expressed.

The above-mentioned genes are generally abundantly expressed in hepatocytes; on the other hand, it is known that many of these genes are not substantially expressed in non-hepatocytes including embryonic stem cells.

In another example of the induced hepatic stem cell described herein, the following genes may be expressed as the genes in (2) above associated with the properties of a hepatocyte.

In the induced hepatic stem cell described herein, GSTM3 gene, SLC22A1 gene, GSTA5 gene, ALDH1A1 gene, CYP27A1 gene, CYP1B1 gene, ALDH2 gene, GSTA2 gene, GSTA3 gene, GSTA5 gene, CYP4A2 gene, UGT2B11 gene, and the like may be expressed. The induced hepatic stem cell expressing these genes displays a property of a hepatocyte that produces proteins associated with drug kinetics, so it is particularly useful in a toxicity test method.

In the induced hepatic stem cell described herein, GSTM3 gene, SLC22A1 gene, GSTA5 gene, ALDH1A1 gene, CYP27A1 gene, CYP1B1 gene, ALDH2 gene, GSTA2 gene, GSTA3 gene, GSTA5 gene, CYP4A2 gene, UGT2B11 gene, and the like may be expressed. The induced hepatic stem cell expressing these genes displays a property of a hepatocyte that produces proteins associated with enzymes associated with drug metabolism, so it is particularly useful in a metabolism test method.

In the induced hepatic stem cell described herein, CD81 gene, SCARB1 gene, OCLN gene, CLDN1 gene, and the like may be expressed. The induced hepatic stem cell expressing these genes displays a property of a hepatocyte that produces proteins associated with the replication of HCV, so it is particularly useful in an antiviral activity test method.

In the induced hepatic stem cell described herein, APOA1 gene, APOA2 gene, APOA4 gene, APOB gene, FABP1 gene, AGT gene, and the like maybe expressed. The induced hepatic stem cell expressing these genes displays a property of a hepatocyte that produces proteins associated with lipid metabolism and blood pressure, so it is particularly useful in a screening test for pharmaceuticals such as hyperlipidemic therapeutics and hypertension therapeutics.

In the induced hepatic stem cell described herein, CCL2 gene, CDKN1A gene, ICAM1 gene, JUNB gene, RGS2 gene, CCND1 gene, and the like may be expressed. The induced hepatic stem cell expressing these genes displays a property of a hepatocyte that produces transporters and metabolic receptor-associated proteins, so it is particularly useful in a screening test for pharmaceuticals such as low-molecular weight compounds and antibodies.

In the induced hepatic stem cell described herein, ALB gene, TTR gene, TF gene, RBP4 gene, FGA gene, FGB gene, FGG gene, AHSG gene, AFP gene, FN1 gene, SERPINA1 gene, PLG gene, and the like may be expressed. The induced hepatic stem cell expressing these genes displays a property of a hepatocyte that produces serum proteins, so it is particularly useful in a method for preparation of animal models.

In the induced hepatic stem cell described herein, ALB gene, TTR gene, TF gene, RBP4 gene, FGA gene, FGB gene, FGG gene, AHSG gene, AFP gene, FN1 gene, SERPINA1 gene, PLG gene, and the like may be expressed. The induced hepatic stem cell expressing these genes displays a property of a hepatocyte that produces serum proteins, so it is particularly useful in a therapeutic method directed at non-human animals.

The induced hepatic stem cell described herein may have properties characteristic of mesendodermal stem cells and/or endodermal stem cells, and they may also have expressed therein at least one of the following genes which are expressed in mesendodermal stem cells and/or endodermal stem cells, namely, SOX17 gene, FOXA2 gene, GSC gene, EOMES gene, and TCF2 gene. A particular example is one that expresses all of SOX17 gene, FOXA2 gene, GSC gene, EOMES gene, and TCF2 gene.

In addition, the induced hepatic stem cell described herein may be such that at least one gene as selected from the genes listed in Table 3 above which are associated with the production of drug metabolizing enzymes and transporters has its expression suppressed or induced, or has the activity of a gene product of said gene promoted or inhibited, by a test substance. The test substance as used herein refers to candidate substances for pharmaceuticals and when the induced hepatic stem cell described herein incorporates such a test substance, the genes associated with the production of drug metabolizing enzymes and transporters are suppressed in or induced for expression in the induced hepatic stem cell described herein and the activity of gene products of these genes is promoted or inhibited. Such cells are useful in drug discovery applications such as a drug metabolism test. The expression of drug metabolism genes including transporter genes and nuclear receptor genes is known to have individual differences. Since the induced hepatic stem cell described herein can be induced from various cells, it is possible to obtain a sufficiently large number of induced stem cells to cover these individual differences. Therefore, if, in the induced hepatic stem cell described herein, the expression of the genes listed in Table 3 above were suppressed or induced, and the activity of gene products of said genes were induced or inhibited, by a test substance, it is useful as a tool for drug discovery. Accordingly, the induced hepatic stem cell described herein is useful in a drug kinetics test, a safety test, a toxicity test, a metabolism test, a drug interaction test, and the like.

Since the induced hepatic stem cell described herein displays various properties of hepatocytes, it is very useful in analyzing the metabolism and mechanism of action of various pharmaceuticals and compounds, as well as searching and analyzing molecules that control the formation and functions of the liver. Hence, it can be used in safety tests, toxicity tests, metabolism tests, drug interaction tests, antiviral activity tests (especially on type B or C hepatitis), screening tests for pharmaceuticals such as hyperlipidemic therapeutics, hypertension therapeutics, low-molecular weight compound medicaments, and antibody medicaments, screening for targets in drug discovery (e.g. hepatic fibrosis, cirrhosis, fatty liver, hepatitis, metabolic syndrome, and hematopoiesis), production of hepatocyte-produced proteins, preparation of animal models, regenerative medicine, and the like.

The induced hepatic stem cell described herein can be subjected to expansion culture or passage culture for at least 3 days. More specifically, an induced hepatic stem cell can be proliferated for at least a month, half a year or even one year and longer; this means that it is theoretically capable of self-replication unlimitedly.

Culture media for expansion culture or passage culture of the induced hepatic stem cell described herein are not particularly limited as long as they permit the expansion culture or passage culture of embryonic stem cells, pluripotent stem cells, and the like; media suitable for the culture of embryonic stem cells, pluripotent stem cells, and the like are preferably used. Examples of such media include, but are not limited to, an ES medium [40% Dulbecco's modified Eagle medium (EMEM), 40% F12 medium (Sigma), 2 mM L-glutamine or GlutaMAX (Sigma), 1% non-essential amino acid (Sigma), 0.1 mM β-mercaptoethanol (Sigma), 15-20% Knockout Serum Replacement (Invitrogen), 10 µg/ml of gentamicin (Invitrogen), and 4-10 ng/ml of FGF2 factor]; a conditioned medium that is the supernatant of a 24-hr culture of mouse embryonic fibroblasts (hereinafter referred to as MEF) on an ES medium lacking 0.1 mM β-mercaptoethanol and which is supplemented with 0.1 mM β-mercaptoethanol and 10 ng/ml of FGF2 (this medium is hereinafter referred to as MEF conditioned ES medium), an optimum medium for iPS cells (iPSellon), an optimum medium for feeder cells (iPSellon), StemPro (registered trademark) hESC SFM (Invitrogen), mTeSR1 (STEMCELL Technologies/VERITAS), an animal protein free, serum-free medium for the maintenance of human ES/iPS cells, named TeSR2 [ST-05860] (STEMCELL Technologies/VERITAS), a medium for primate ES/iPS cells (ReproCELL), ReproStem (ReproCELL), and ReproFF (ReproCELL). For human cells, media suitable for culturing human embryonic stem cells may be used.

The techniques for effecting expansion culture or passage culture of the induced hepatic stem cell described herein are not particularly limited if they are methods commonly used by the skilled artisan to culture embryonic stem cells, pluripotent stem cells, and the like. For example, after removing culture medium from the cultured cells and wasihing the cells with PBS(-), a dissociation solution is added and after standing for a given period, the dissociation solution is removed and after adding a D-MEM (high glucose) medium supplemented with 1X antibiotic/antimycotic and 10% FBS, centrifugation is performed and the supernatant is removed; thereafter, 1X antibiotic/antimycotic, mTeSR and Y-27632 are added and the cell suspension is seeded on an MEF-seeded gelatin- or collagen-coated dish for effecting passage culture.

In order to ensure that the induced hepatic stem cell described herein will not differentiate even if it is cultured for longer than a month after gene transfer, various inhibitors or antibodies that will inhibit or neutralize the activity of TGF-beta and the like, HGF, fibroblast growth factors such as FGF1 - FGF21, activin and the like may be added to the medium; fibroblast growth factors that are preferably used include the acidic fibroblast growth factor FGF1 (also called aFGF and hereinafter designated as FGF1), as well as the basic fibroblast growth factor FGF2 (also called bFGF and hereinafter designated as FGF2), FGF4, and FGF7. Exemplary antibodies are polyclonal or monoclonal neutralizing antibodies against these growth factors. If desired, microRNAs, siRNAs and antisense RNAs may be used to suppress the expression of genes such as TGF-beta. It is also possible to use inhibitors as low-molecular weight compounds that act against TGF-beta and the like. Exemplary TGF-beta signaling inhibitors include an ALK inhibitor (e.g. A-83-01), a TGF-beta RI inhibitor, and a TGF-beta RI kinase inhibitor. It should be noted that the above-mentioned fibroblast growth factors are selected depending on the type of the somatic cell to be induced and there can be used fibroblast growth factors derived from human, mouse, cow, horse, pig, zebrafish, etc.

Furthermore, inhibitors of Rho associated kinase (Rho-associated coiled coil containing protein kinase), such as Y-27632 (Calbiochem; water soluble) and Fasudil (HA1077:Calbiochem) can also be added to the medium.

Other inhibitors that can be added to the medium include: three low-molecular weight inhibitors of FGF receptor tyrosine kinase, MEK (mitogen activated protein kinase)/ERK (extracellular signal regulated kinases 1 and 2) pathway, and GSK (Glycogen Synthase Kinase) 3 [SU5402, PD184352, and CHIR99021], two low-molecular weight inhibitors of MEK/ERK pathway and GSK3 [PD0325901 and CHIR99021], a low-molecular weight compound as an inhibitor of the histone methylating enzyme G9a [BIX-01294 (BIX)], azacitidine, trichostatin A (TSA), 7-hydroxyflavone, lysergic acid ethylamide, kenpaullone, an inhibitor of TGF-β receptor I kinase/activin-like kinase 5 (ALK5) [EMD 616452], inhibitors of TGF-β receptor 1 (TGFBR1) kinase [E-616452 and E-616451], an inhibitor of Src-family kinase [EI-275], thiazovivin, PD0325901, CHIR99021, SU5402, PD184352, SB431542, anti-TGF-β neutralizing antibody, A-83-01, Nr5a2, a p53 inhibiting compound, siRNA against p53, an inhibitor of p53 pathway, etc.

In addition, the induced hepatic stem cell described herein can be frozen or thawed by known methods. An exemplary method of freezing that may be used is the following: after removing culture medium from the cultured cells and washing the cells with PBS(-), a dissociation solution is added and after standing for a given period, the dissociation solution is removed and after adding a D-MEM (high glucose) medium supplemented with 1X antibiotic/antimycotic and 10% FBS, centrifugation is performed and the supernatant is removed; thereafter, a cryopreservation fluid is added and the mixture is distributed into cryogenic vials, frozen overnight at -80 °C and thereafter stored in liquid nitrogen. An exemplary method of thawing is the following: the frozen sample is thawed in a water bath with 37 °C and then suspended in a D-MEM (high glucose) medium supplemented with 1X antibiotic/antimycotic and 10% FBS before use.

A second aspect described herein relates to a process for producing an induced hepatic stem cell comprising a step of inducing a mammalian cell to an induced hepatic stem cell, and the mammal to be treated is not particularly limited as long as it is a mammal and may be exemplified by rat, mouse, guinea pig, rabbit, dog, cat, pig such as minipig, cow, horse, primates such as monkeys including a cynomolgus, and human, with rat, mouse, guinea pig, dog, cat, minipig, horse, cynomolgus, and human being preferred, and human is used with particular preference.

Any of the cells of the above-mentioned mammals may be used as long as they are mammalian cells. Examples that may be used include but are not limited to cells of organs such as the brain, liver, esophagus, stomach, duodenum, small intestine, large intestine, colon, pancreas, kidney, and lung, as well as cells of bone marrow fluid, muscle, fat tissue, peripheral blood, skin, and skeletal muscle. Among these, cells derived from endodermal liver, stomach, duodenum, small intestine, large intestine, colon, pancreas, lung, etc. are preferred, with cells derived from the stomach and colon being used with particular preference. These cells also are preferred in that they are readily available as medical waste during operation in cancer therapy.

It is also possible to use cells derived from tissues and body fluids that accompany childbirth such as cells derived from umbilical cord tissues (umbilical cord and umbilical blood), amnion, placenta and amniotic fluid; in particular, there may be used cells derived from tissues just after birth such as various tissues of neonates (e.g., neonatal skin). It is also possible to use cells of fetal animals.

The cells of the above-mentioned mammals that may be used include adult-derived cells, neonate-derived cells, neonatal skin-derived cells, cancerous individual's cells, embryonic stem cells, induced pluripotent stem cells, and cells differentiated from embryonic stem cells or induced pluripotent stem cells.

The types of cancers in cancerous individuals are not particularly limited and any of cancers such as malignant tumor, solid cancer, carcinoma, sarcoma, brain tumor, hematopoietic organ cancer, leukemia, lymphoma, and multiple myeloma can be used. Examples include, but are not limited to, oral cancer, cancer of the throat, cancer of upper airway, lung cancer, lung cell cancer, esophageal cancer, stomach cancer, duodenal cancer, pancreatic cancer, liver cancer, gallbladder cancer, biliary tract cancer, bowel cancer, colon cancer, rectal cancer, breast cancer, thyroid cancer, uterine body cancer, cervical cancer, ovary cancer, testis cancer, kidney cancer, bladder cancer, prostate cancer, skin cancer, malignant melanoma, brain tumor, bone sarcoma, and blood cancer. Among others, cells derived from non-cancer or cancer tissues in individuals with endodermal stomach, breast, colon and bowel cancers are preferably used.

In the production process described herein, cells harvested from mammals may also be used as the above-mentioned mammalian cells. Cells harvested from mammals may immediately used or they can be used after being stored and cultured by known methods. In the case of culturing, the number of passages is not particularly limited but cells from a primary culture to a fourth passage culture are preferred, with the use of cells from a primary culture to a second passage culture being particularly preferred. The primary culture as used herein means a culture that immediately follows a harvest of cells from mammals and one passage culture of the primary culture results in a second passage culture and one more passage culture results in a third passage culture.

The step of inducing a mammalian cell to an induced hepatic stem cell in the production process described herein must be a step in which the mammalian cell is brought to such a state that gene products of POU5F1 gene, KLF4 gene, and SOX2 gene which are necessary for induction to the induced hepatic stem cell will be present to ensure that the intracellular relative abundance of the gene product of POU5F1 gene is greater than that of the gene product of SOX2 gene. The term "bringing the mammal cell to such a state" is a broad concept that includes not only the case of adjusting the cell to have such a state but also the case of selecting a cell that has been brought to such a state and conditioning the same.

The production process described herein also requires that gene products of those genes should be present in specified proportions within the mammalian cell as it is induced to give rise to the induced hepatic stem cell describd herein. If this condition is applied, the marker genes for the embryonic stem cell in (1) above that are endogenous to the mammalian cell are expressed, eventually giving rise to the induced hepatic stem cell described herein.

In the production process described herein, the intracellular relative abundances of the gene products of POU5F1 gene, KLF4 gene, and SOX2 gene which are necessary for induction to the foregoing induced hepatic stem cell may satisfy the relation of POU5F1 gene > KLF4 gene > SOX2 gene, and from the viewpoint of highly efficient induction to the induced hepatic stem cell, the intracellular relative abundances of POU5F1 gene, KLF4 gene, and SOX2 gene may be adjusted to the ratio of 4:2:1 in that order.

In the production process described herein, the foregoing mammalian cell suffices to be brought to such a state that gene products of POU5F1 gene, KLF4 gene, and SOX2 gene which are necessary for induction to the induced hepatic stem cell will be present to ensure that the intracellular relative abundance of the gene product of POU5F1 gene is greater than that of the gene product of SOX2 gene; methods for doing this are exemplified by but are not limited to those which are known as induction techniques for giving rise to induced pluripotent stem cells.

Exemplary methods that may be employed include a method in which genes capable of elevating the intensity of expression of POU5F1 gene, KLF4 gene, and SOX2 gene which are necessary for induction to the foregoing induced hepatic stem cell are introduced into the foregoing mammalian cell, whereby these genes are strongly expressed so that the intended gene products will be produced in the cell, as well as a method in which proteins, mRNAs or the like that are gene products of the genes capable of elevating the intensity of expression of the above-identified genes are introduced into the foregoing mammalian cell. When needed, the amounts of vectors or genes to be introduced into the foregoing mammalian cell, the amounts of gene products to be added to media, and other factors may be so adjusted as to ensure that the intracellular relative abundance of the gene product of POU5F1 gene is greater than that of the gene product of SOX2 gene.

In the production process described herein, genes that may be used to elevate the intensity of expression of POU5F1 gene, KLF4 gene, and SOX2 gene which are necessary for induction to the foregoing induced hepatic stem cell are POU5F1 gene, KLF4 gene, and SOX2 gene per se. If the above-mentioned POUF1 gene, KLF4 gene, or SOX2 gene is expressed only in insufficient amount in the foregoing mammalian cell, the insufficient gene or gene product may be introduced into the same cell, and if the above-mentioned POU5F1 gene, KLF4 gene, or SOX2 gene is expressed in the foregoing cell, other gene or a gene product thereof may be introduced in place of the above-mentioned POU5F1 gene, KLF4 gene, or SOX2 gene. Genes that can be used as such other gene are those that are known to induce induced pluripotent stem cells and they may be exemplified by NANOG gene, LIN28 gene, TBX3 gene, PRDM14 gene, L-MYC gene, c-MYC gene, N-MYC gene, SALL1 gene, SALL4 gene, UTF1 gene, ESRRB gene, NR5A2 gene, REM2 GTPase gene, TCL-1A gene, Yes-associated protein (YAP) gene, E-cadherin gene, p53 dominant negative mutant gene, p53shRNA gene, etc. The genes capable of elevating the intensity of expression of POU5F1 gene, KLF4 gene, and SOX2 gene which are necessary for induction to the foregoing induced hepatic stem cell may be used either independently or in combination of two or more kinds.

POUF1 gene, KLF4 gene, and SOX2 gene which are necessary for induction to the foregoing induced hepatic stem cell may be used in combination with genes that are substitutes for these genes.

For example, in the case where a cell that strongly expresses POU5F1 gene, KLF4 gene, c-MYC gene, or SOX2 gene, the induced hepatic stem cell described herein can be induced without using POU5F1 gene, KLF4 gene, c-MYC gene, or SOX2 gene but by using p53 dominant negative mutant gene, p53shRNA gene, etc. in combination.

Methods by which proteins, mRNAs or the like that are gene products of POU5F1 gene, KLF4 gene, and SOX2 gene which are necessary for induction to the foregoing induced hepatic stem cell or genes that are substitutes for these genes can be introduced into the foregoing mammal cell include, but are not limited to, those which are known as induction techniques for giving rise to induced pluripotent stem cells. For example, proteins, mRNAs or the like that are gene products of these genes may be added to media.

In the production process described herein, in order to increase the efficiency of induction to the induced hepatic stem cell, compounds that are known to induce induced pluripotent stem cells may further be added to the media used to induce the induced hepatic stem cell described herein, and these compounds are exemplified by inhibitors including: three low-molecular weight inhibitors of FGF receptor tyrosine kinase, MEK (mitogen activated protein kinase)/ERK (extracellular signal regulated kinases 1 and 2) pathway, and GSK (Glycogen Synthase Kinase) 3 [SU5402, PD184352, and CHIR99021], two low-molecular weight inhibitors of MEK/ERK pathway and GSK3 [PD0325901 and CHIR99021], a low-molecular weight compound as an inhibitor of the histone methylating enzyme G9a [BIX-01294 (BIX)], azacitidine, trichostatin A (TSA), 7-hydroxyflavone, lysergic acid ethylamide, kenpaullone, an inhibitor of TGF-β receptor I kinase/activin-like kinase 5 (ALK5) [EMD 616452], inhibitors of TGF-β receptor 1 (TGFBR1) kinase [E-616452 and E-616451], an inhibitor of Src-family kinase [EI-275], thiazovivin, PD0325901, CHIR99021, SU5402, PD184352, SB431542, anti-TGF-β neutralizing antibody, A-83-01, Nr5a2, a p53 inhibiting compound, siRNA against p53, and an inhibitor of p53 pathway, ctc.

It is also possible to use a microRNA to increase the efficiency of induction to the induced hepatic stem cell. Specifically, common methods for the skilled artisan may be carried out, as by introducing a microRNA into the foregoing mammalian cell with a vector or adding a microRNA to the medium.

Examples of the microRNA that can be used to increase the efficiency of induction to the induced hepatic stem cell include miR-154, miR-200, miR-368, miR-371, miR-291-3p, miR-294, miR-295, miR-302, etc. If a human cell is used as the mammaian cell, a human microRNA may be used. Specific examples include, but are not limited to, hsa-miR-372 [MI0000780], hsa-miR-373 [MI0000781], hsa-miR-302b [MI0000772], hsa-miR-302c [MI0000773], hsa-miR-302a [MI0000738], hsa-miR-302d [MI0000774], hsa-miR-367 [MI0000775], and hsa-miR-520 [MI0003158]. These microRNAs may be used either independently or in combination of two or more kinds.

Information about these microRNAs can be accessed from the web site of miRBase (http://www.mirbase.org/). In each designation, a miRBase accession number is parenthesized and the symbol hsa- represents human.

The step of inducing the foregoing mammalian cell to an induced hepatic stem cell may involve the use of various inhibitors or antibodies that will inhibit or neutralize the activity of TGF-beta and the like, fibroblast growth factors such as FGF1 - FGF21, and the like, which are to be added to the medium for culturing the induced hepatic stem cell described herein. Fibroblast growth factors that may be used are FGF1, FGF2, FGF4, and FGF7. Exemplary TGF-beta inhibitors include TGF-beta signaling inhibitors such as an ALK inhibitor (e.g. A-83-01), a TGF-beta RI inhibitor, and a TGF-beta RI kinase inhibitor.

These components are preferably added to the medium to be used in the step of inducing the foregoing mammalian cell to an induced hepatic stem cell.

The above-mentioned step for induction to an induced hepatic stem cell may be such that it uses POU5F1 gene, KLF4 gene, and SOX2 gene which are necessary for induction to the induced hepatic stem cell or gene products of these genes, and that the ratio in use of POUF1 gene or a gene product of this gene to SOX2 gene or a gene product of this gene is greater than one. The ratio in use between POU5F1 gene, KLF4 gene, and SOX2 gene which are necessary for induction to the induced hepatic stem cell or between gene products of these genes may satisfy the relation of POU5F1 gene > KLF4 gene > SOX2 gene, and from the viewpoint of highly efficient induction to the induced hepatic stem cell, the ratio in use between POUF1 gene, KLF4 gene, and SOX2 gene or between gene products of these genes may be 4:2:1 in that order. The gene symbols for POU5F1 (OCT3/4) gene, KLF4 gene, and SOX2 gene, as well as the corresponding Genbank accession numbers are given in Table 4.

**[Table 4]**

| GeneSymbol | GenbankAccession |
|---|---|
| KLF4 | NM_004235 |
| POU5F1 | NM_002701 |
| SOX2 | NM_003106 |

If POU5F1 gene, KLF4 gene, and SOX2 gene which are necessary for induction to the foregoing induced hepatic stem cell are used in the production process described herein, common methods for the skilled artisan may be used, as by introducing these genes into the foregoing mammalian cell with the aid of expression vectors. If gene products such as proteins or mRNAs of the foregoing POU5F1 gene, KLF4 gene, and SOX2 gene which are necessary for induction to the induced hepatic stem cell are used, common methods for the skilled artisan may be used, as by adding the gene products to the medium used for induction.

As described above, in addition to POU5F1 gene, KLF4 gene, and SOX2 gene which are necessary for induction to the foregoing induced hepatic stem cell, as well as the gene products thereof, the following which have been noted above may typically be used in order to enhance the efficiency of induction to the foregoing induced hepatic stem cell: genes such as NANOG gene, LIN28 gene, TBX3 gene, PRDM14 gene, L-MYC gene, c-MYC gene, N-MYC gene, SALL1 gene, SALL4 gene, UTF1 gene, ESRRB gene, NR5A2 gene, REM2 GTPase gene, TCL-1A gene, Yes-associated protein (YAP) gene, E-cadherin gene, p53 dominant negative mutant gene, p53shRNA gene, as well as gene products and compounds thereof; fibroblast growth factors such as FGF1 to FGF12; as well as ALK inhibitor (e.g. A-83-01), TGF-beta RI inhibitor, and TGF-beta RI kinase inhibitor.

To produce induced hepatic stem cells from the foregoing mammalian cell, genes may be introduced into the foregoing mammalian cell by any known methods without particular limitation, and vectors that can be used include viral vectors, plasmids, artificial chromosomes (HAC), episomal vectors (EBV), minicircle vectors, polycistronic expression vectors, vectors as an application of the Cre/loxP system, vectors making use of a phage integrase, and a transposon such as a piggyback.

Viral vectors that can be used to introduce genes into the foregoing mammalian cell may be of any known types. Examples include, but are not limited to, lentiviral vectors, retroviral vectors, adenoviral vectors, simian immunodeficiency virus vectors (DNAVC Corporation), adeno-associated viral vectors (DNAVC Corporation), Sendai virus vectors having no residual exogenous genes in the genome (DNAVC Corporation, and MEDICAL & BIOLOGICAL LABORATORIES CO., LTD.), Sendai mini vectors (DNAVC Corporation), and HVJ. Retroviral vectors include Moloney murine leukemia derived retroviral vectors.

Viral vector plasmids that can be used may be of any known types of viral vector plasmids. For example, as retroviral vector plasmids, preferred are pMXs, pMXs-IB, pMXs-puro, and pMXs-neo (pMXs-IB being the same vector as pMXs-puro except that it carries a blasticidin resistance gene instead of the puromycin resistance gene) [Toshio Kitamura et. al., "Retrovirus-mediated gene transfer and expression cloning: Powerful tools in functional genomics", Experimental Hematology, 2003, 31(11):1007-14], and other examples include MFG [Proc. Natl. Acad. Sci. USA, 92, 6733-6737 (1995)], pBabePuro [Nucleic Acids Research, 18, 3587-3596 (1990)], LL-CG, CL-CG, CS-CG, CLG [Journal of Virology, 72, 8150-8157 (1998)], etc. Adenoviral vector plasmids include pAdex1 [Nucleic Acids Res., 23, 3816-3821 (1995)], etc.

Media that can be used in the step of inducing the foregoing mammalian cell to induced hepatic stem cells are not limited to any particular types as long as they permit culturing embryonic stem cells, pluripotent stem cells, and the like, but culturing may be performed using media suitable for culturing embryonic stem cells, pluripotent stem cells, and the like. Examples of such media include, but are not limited to, an ES medium, an MEF conditioned ES medium, an optimum medium for iPS cells, an optimum medium for feeder cells, StemPro (registered trademark) hESC SFM, mTeSR1, an animal protein free, serum-free medium for the maintenance of human ES/iPS cells, named TeSR2 [ST-05860], a medium for primate ES/iPS cells, ReproStem, and ReproFF. For human cells, media suitable for culturing human embryonic stem cells are preferably used.

If the derived cell is not a fibroblast, for example, in the case of using an epithelial cell such as one derived from a patient with stomach or colon cancer, it is preferably co-cultured with a feeder cell after gene transfer.

A third aspect described herein relates to a test method characterized by using the induced hepatic stem cell described herein. The test method described herein can advantageously be used as a safety test method, a toxicity test method, a metabolism test method, a drug interaction test method, an antiviral activity test method, or a screening test method for pharmaceuticals such as hyperlipidemic therapeutics, hypertension therapeutics, low-molecular weight compound medicaments, and antibody medicaments.

In an exemplary drug interaction test method that uses the induced hepatic stem cell described herein, human induced hepatic stem cells are prepared from donors of different races, sexes, ages, genetic backgrounds (e.g. polymorphisms), etc., cultured with a pharmaceutical candidate compound, and the expression of genes for various enzymes in cytochrome P450 (CYP) subfamilies in these cells is examined using DNA microarrays (KURABO INDUSTRIES LTD.) or multifunctional gene expresser GenomeLabTM GeXP (Beckman Coulter, Inc.) to thereby reveal the interaction between each of the cytochrome P450 (CYP) subfamily enzymes and the pharmaceutical candidate compound tested. The interaction between a cytochrome P450 (CYP) subfamily enzyme and a pharmaceutical candidate compound can also be examined by a method of using a substrate that produces a fluorescence product after it is metabolized by a cytochrome P450 enzyme.

Cytochrome P450 enzymes are important catalysts that oxidatively metabolize a broad range of hydrophobic chemical substances and since the drug metabolism by these enzymes is involved in drug clearance, toxicity, and activation, they are known to potentially have influence on harmful interactions between drugs. Hence, development of low-molecular weight therapeutics and the like requires a close study of the enzyme-drug interaction.

In an exemplary antiviral activity test method that uses the induced hepatic stem cell described herein, a culture medium in which the induced hepatic stem cell described herein is being cultured is infected with added hepatitis A, B or C virus and then a pharmaceutical candidate compound for an antiviral drug is added for evaluation of its efficacy.

In an exemplary hyperlipidemic therapeutic screening test method that uses the induced hepatic stem cell described herein, a hyperlipidemic therapeutic candidate compound is added to a plate on which the induced hepatic stem cell described herein is being cultured and after continued culture, lipoproteins and lipids secreted into the culture supernatant are analyzed to evaluate the efficacy of the added hyperlipidemic therapeutic candidate compound.

In an exemplary test method for analyzing the lipoproteins and lipids secreted into the culture supernatant, proteins such as CM (chylomicrons), VLDL (very low-density lipoprotein), LDL (low-density lipoprotein), and HDL (high-density lipoprotein), as well as lipids such as FC (free cholesterol), PL (phospholipids), and TC (total cholesterol) are analyzed by gel permeation HPLC (LipoSEARCH; Skylight Biotech, Inc.)

Measurements are also possible by the methods described in Usui S, Hara Y, Hosaki S, Okazaki M, "A new on-line dual enzymatic method for simultaneous quantification of cholesterol and triglycerides in lipoproteins by HPLC", J. Lipid Res., 2002;43:805-14, and Mitsuyo Okazaki, Shinichi Usui, Masato Ishigami, Naohiko Sakai, Tadashi Nakamura, Yuji Matsuzawa, Shizuya Yamashita, "Identification of Unique Lipoprotein Subclasses for Visceral Obesity by Component Analysis of Cholesterol Profile in High-Performance Liquid Chromatography", Arterioscler Thromb Vasc Biol. March 2005.

A fourth aspect described herein relates to a method of screening for targets in drug discovery that is characterized by using the induced hepatic stem cell described herein.

A fifth aspect described herein relates to a method for preparation of animal models that is characterized by using the induced hepatic stem cell described herein.

A sixth aspect described herein relates to a method for production of hepatocyte-produced proteins that is characterized by characterized by using the induced hepatic stem cell described herein.

The induced hepatic stem cell described herein has properties of a hepatocyte, so it can produce proteins characteristic of various hepatocytes. Hence, an exemplary method according to the sixth aspect described herein comprises culturing an induced hepatic stem cell described herein that produces a protein specific for a particular hepatocyte and producing a protein characteristic of that hepatocyte.

A seventh aspect described herein relates to a therapeutic method directed to mammals that is characterized by characterized by using the induced hepatic stem cell described herein.

In the therapeutic method described herein, the induced hepatic stem cell described herein as induced from a mammalian cell may be transplanted in the liver of the mammal. For example, the induced hepatic stem cell described herein as induced from a canine cell can be transplanted in the liver of the dog.

The present invention is illustrated more specifically by means of the following Examples but it should be understood that the scope of the present invention is by no means limited by those Examples.

### EXAMPLE 1

### 1. Preparation of a pantropic retroviral vector

Three retroviral vector plasmids for three genes, POU5F1-pMXs, KLF4-pMXs, and SOX2-pMXs, were introduced into packaging cells for preparing a pantropic retroviral vector, Plat-GP cells, using Fugene HD (Roche; Cat No. 4709691) to thereby prepare a retroviral vector solution. The vector plasmids POU5F1-pMXs, KLF4-pMXs, and SOX2-pMXs were used at a ratio of 4:2:1 in that order. The ratio of 4:2:1 may be achieved when the genes are introduced into packaging cells or may be achieved by preparing separate retroviral vector solutions for POU5F1-pMXs, KLF4-pMXs, and SOX2-pMXs, and mixing these solutions at a ratio of 4:2:1 in that order. The details of the procedure are as described below.

### <Preparation of a retroviral vector solution for introducing the genes into cells derived from neonatal skin tissues>

The vectors POU5F1-pMXs, KLF4-pMXs, and SOX2-pMXs were supplied by Addgene (Table 5 below).

The amounts of the respective vectors were as follows: 2 µg of POU5F1-pMXs (Addgene), 1 µg of KLF4-pMXs (Addgene), 0.5 µg of SOX2-pMXs (Addgene), 0.5 µg of Venus-pCS2 (Nagai T et al. A variant of yellow fluorescent protein with fast and efficient maturation for cell-biological applications. Nat Biotechnol 2002; 20: 87-90), 2 µg of VSV-G-pCMV (Cell Biolab), and 18 µL of FuGENE HD (Roche).

### <Preparation of a retroviral vector solution for introducing the genes into cells derived from stomach cancer patient's cancer tissues>

The vectors POU5F1-pMXs, KLF4-pMXs, and SOX2-pMXs were constructed vectors (Table 5).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs (Cell Biolab), and 45 µL of FuGENE HD.

<Preparation of a retroviral vector solution for introducing the genes into cells derived from stomach cancer patient's non-cancer tissues>

The vectors POU5F1-pMXs, KLF4-pMXs, and SOX2-pMXs were constructed vectors (Table 5).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs, and 45 µL of FuGENE HD.

### <Preparation of a retroviral vector solution for introducing the genes into cells derived from adult skin tissues>

The vectors POU5F1-pMXs, KLF4-pMXs, and SOX2-pMXs were constructed vectors (Table 5).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs, and 45 µL of FuGENE HD.

### <Preparation of a retroviral vector solution for introducing the genes into cells derived from colon cancer patient's cancer tissues>

The vectors POU5F1-pMXs, KLF4-pMXs, and SOX2-pMXs were constructed vectors (Table 5).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs, and 45 µL of FuGENE HD.

Plat-GP cells into which the retroviral vector plasmids had been introduced were cultured for at least 48 hours; thereafter, the supernatant was harvested three times every 24 hours, and filtration was performed using the Steriflip-HV Filter unit (pore size 0.45 µm filter; Millipore; Cat No. SE1M003M00). The above-noted procedure yielded a pantropic retroviral vector solution containing the three genes (POU5F1, KLF4, and SOX2 at a ratio of 4:2:1 in that order). The pantropic retroviral vector, which enables gene transfection into various cells, efficiently introduced the genes into human cells as well.

**[Table 5]**

| Details of constructed retroviral vector plasmids and those distributed by Addgene | | | | | | |
|---|---|---|---|---|---|---|
| Gene | NCBI No. | Vector | 5' restriction enzyme | 3' restriction enzyme | Clone ID | Supplier |
| Human POU5F1 | BC117435 | pMXs | EcoRI | EcoRI | 40125986 | Open Biosystems |
| Human KLF4 | BC029923 | pMXs | EcoRI | EcoRI | 5111134 | Open Biosystems |
| Human SOX2 | BC013923 | pMXs | EcoRI | XhoI | 2823424 | Open Biosystems |
| Human POU5F1 | RT-PCR & Cloning | pMXs | EcoR1 | EcoR1 | 17217 | Addgene |
| Human KLF4 | RT-PCR & Cloning | pMXs | attB1 | attB2 | 17219 | Addgene |
| Human SOX2 | RT-PCR & Cloning | pMXs | attB1 | attB2 | 17218 | Addgene |

### EXAMPLE 2

### 2. Preparation of induced human hepatic stem cells from cells derived from neonatal skin tissues

Induced human hepatic stem cells were prepared from cells derived from neonatal human skin tissues which is postpartum tissues (trade name: normal neonatal human skin fibroblasts; primary culture; Lot No. 7F3956).

One vial of cryopreserved cells derived from neonatal human skin tissues (primary culture; Lonza; CC-2511; Lot No. 7F3956) was thawed in a water bath at 37°C and suspended in a D-MEM (high glucose) (Invitrogen; Cat No. 11965-092) medium supplemented with 1X antibiotic/antimycotic (Invitrogen; Cat No. 15240-062) and 10% FBS to thereby obtain 10 mL of a cell suspension.

Then, the obtained cell suspension was centrifuged at 1000 rpm at 4°C for 5 minutes to remove the supernatant, and thereafter the remaining cells were resuspended in 12 mL of Fibroblast Growth Medium Kit-2 (2% FBS) (hereinafter referred to as FGM-2 BulletKit™) (Lonza; Cat No. CC-3132) to thereby obtain a cell suspension. The obtained cell suspension was added at a volume of 2 mL per well onto a 6-well plastic plate (Nunc; Cat No. 140675) whose well bottoms had been coated with matrigel (Becton, Dickinson; Cat No. 356230) at a concentration of 20 µg/cm² for at least 30 minutes, whereby cells were seeded.

After 3 days, the medium was removed, and a retroviral vector solution containing the three genes (POU5F1, KLF4, and SOX2 at a ratio of 4:2:1 in that order) was added in a volume of 2 mL per well to allow infection to proceed at 37°C for 24 hours. After removal of the viral supernatant, FGM-2 BulletKit was added in a volume of 2 mL per well and cells were cultured at 37°C for one day. Then, a MEF conditioned ES medium was repeatedly replaced every two days, and an ES medium was replaced on 12, 14 and 17 days after the introduction of the three genes. The formulations of the MEF conditioned ES medium and ES medium used were as follows.

### <MEF conditioned ES medium>

### MEF

Mitomycin C-treated primary mouse embryonic fibroblasts [DS Pharma Biomedical] Cat No. R-PMEF-CF

### Conditioned ES medium

Knockout D-MEM (Invitrogen; Cat No. 10829-018), 500 mL
20% knockout serum replacement (Invitrogen; Cat No. 10828-028)
50 µg/mL gentamicin (Invitrogen; Cat No. 15750-060)
1X MEM non-essential amino acid solution (Invitrogen; Cat No. 11140-050)
10 ng/mL bFGF (PeproTech; Cat No. 100-18B)
0.1 mM 2-Mercaptoethanol (Sigma-Aldrich; Cat No. M7154)
2 mM GlutaMAX or 2 mM L-glutamine

### <ES medium>

Knockout D-MEM (Invitrogen; Cat No. 10829-018), 500 mL
20% knockout serum replacement (Invitrogen; Cat No. 10828-028)
50 µg/mL gentamicin (Invitrogen; Cat No. 15750-060)
1X MEM non-essential amino acid solution (Invitrogen; Cat No. 11140-050)
10 ng/mL bFGF (PeproTech; Cat No. 100-18B)
10³ U/mL recombinant human LIF (Wako Pure Chemical; Cat No. 129-05601)
0.1 mM 2-Mercaptoethanol (Sigma-Aldrich; Cat No. M7154)
0.5 µM ALK5 inhibitor (A-83-01) (Sigma-Aldrich; Cat No. A5480)
0.5 µM PD0325901 (Axon Medchem; Cat No. 1408)
3 µM CHIR99021 (Axon Medchem; Cat No. 1386)

From 18 days after the gene transfection, a feeder-free maintenance medium for human ES/iPS cells, mTeSR1 (STEMCELL Technologies; Cat No. 05850) was replaced everyday. Thirty days after the gene transfection, one clone of a cell colony (NFB1-3) was picked up with forceps and transferred onto feeder cells. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² on the day before the pickup of induced hepatic stem cells.

Listed below are the passage numbers (p) of induced human hepatic stem cells derived from neonatal skin tissues, and the days when they were subjected to passage culture and lysed in a buffer for an RNA collection kit.

### <Induced human hepatic stem cells derived from neonatal skin tissues>

### NFB1-3

Day 52: 24-well (p1) → 6-well (p2)
Day 55: 6-well (p2) → 10 cm (p3)
Day 61: Passage (p4)
Day 62: Treatment with a buffer RLT (solution for lysing cells before RNA purification) (p5)

### EXAMPLE 3

### 3. Preparation of induced human hepatic stem cells from cells derived from cancer tissues of a stomach cancer patient

Cells were isolated from cancer tissues of a patient with (progressive) stomach cancer. To the obtained cells, a retroviral vector solution containing the three genes (POU5F1, KLF4, and SOX2 at a ratio of 4:2:1 in that order) was added for gene transfection to thereby prepare induced human hepatic stem cells. The details of the procedure are as described below.

Part of fresh stomach cancer tissues obtained during operation (from a 67-year-old Japanese male patient with developed cancer) was washed with Hank's balanced salt solution (Phenol Red-free) (Invitrogen; Cat No. 14175-095) and minced with scissors into pieces of about 1 mm². The pieces were further washed with Hank's balanced salt solution (Phenol Red-free) until a transparent supernatant was obtained. After removal of the supernatant, 5 mL of a mixture of 0.01% collagenase (Wako Pure Chemical; Cat No. 034-10533) and 1X antibiotic/antimycotic (Invitrogen; Cat No. 15240-062) was added to the tissue precipitate, and stirring was performed at 37°C for 60 minutes with a shaker.

After the tissue precipitate was confirmed to have been fully digested, 35 mL of a D-MEM (high glucose) (Invitrogen; Cat No. 11965-092) medium supplemented with 1X antibiotic/antimycotic and 10% FBS was added, which was then centrifugated at 1000 rpm at 4°C for 5 minutes. Next, after removal of the supernatant, 40 mL of a D-MEM (high glucose) (Invitrogen; Cat No. 11965-092) medium supplemented with 1X antibiotic/antimycotic and 10% FBS was added, which was then centrifugated performed again at 1000 rpm at 4°C for 5 minutes. Then, after removal of the supernatant, 10 mL of a D-MEM (high glucose) medium supplemented with 1X antibiotic/antimycotic and 10% FBS was added, which was then seeded on a collagen-coated dish (60 mm) (Iwaki; Cat No. 11-018-004).

After 24 hours, the medium was removed, 5 mL of a retroviral vector solution containing the three genes was added, and infected at 37°C for a day. The viral supernatant was removed, and mitomycin treated mouse embryonic fibroblasts (DS Pharma Biomedical; Cat No. R-PMEF-CF) was suspended at a density of 5.0×10⁴ cells/cm² in 5 mL of a D-MEM (high glucose) medium supplemented with 1X antibiotic/antimycotic (Invitrogen; Cat No. 15240-062) and 10% FBS; thereafter, which was seeded a collagen-coated dish (60 mm) (Iwaki; Cat No. 11-018-004) on which the transfected cells derived from the cancer tissues of the stomach cancer patient had been cultured, and co-culturing was performed.

Thereafter, a MEF conditioned ES medium was repeatedly replaced every three days, and from 15 days after the gene transfection, a feeder-free maintenance medium for human ES/iPS cells, mTeSR1 (STEMCELL Technologies; Cat No. 05850) was replaced everyday.

Twenty-five days after the introduction of the three genes, one clone of an induced hepatic stem cell colony (GC1-2) was picked up and subjected to passage culture on mitomycin treated mouse embryonic fibroblasts in a gelatin-coated 24-well plate. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² the day before the pickup of induced hepatic stem cells.

Listed below are the passage numbers (p) of nduced human hepatic stem cells derived from cancer tissues of a stomach cancer patient, and the days when they were subjected to passage culture and lysed in a buffer for an RNA collection kit.

### <Induced human hepatic stem cells derived from cancer tissues of a stomach cancer patient>

### GC1-2

Day 37: 24-well (p1) → 6-well (p2)
Day 43: 6-well (p2) → 10 cm (p3)
Day 56: Passage (p4)
Day 57: Passage and stock (p5)
Day 60: Treatment with a buffer RLT (solution for lysing cells before RNA purification)

### EXAMPLE 4

### 4. Preparation of induced human hepatic stem cells from cells derived from non-cancer tissues of a stomach cancer patient

To cells derived from non-cancer tissues of a stomach cancer patient, a retroviral vector solution containing the three genes (POU5F1, KLF4, and SOX2 at a ratio of 4:2:1 in that order) was added for gene transfection to thereby prepare induced human hepatic stem cells. The details of the procedure are as described below.

Part of fresh non-cancer tissues obtained from a stomach cancer patient during operation (a 67-year-old Japanese male patient with progressive cancer) was washed with Hank's balanced salt solution (Phenol Red-free) (Invitrogen; Cat No. 14175-095) and minced with scissors into pieces of about 1 mm². The pieces were washed with Hank's balanced salt solution (Phenol Red-free) until a transparent supernatant was obtained. Thereafter, the supernatant was removed, 5 mL of a mixture of 0.1% collagenase (Wako Pure Chemical; Cat No. 034-10533) and 1X antibiotic/antimycotic (Invitrogen; Cat No. 15240-062) was added to the tissue precipitate, and stirring was performed at 37°C for 60 minutes with a shaker.

After the tissue precipitate was confirmed to have been fully digested, 35 mL of a D-MEM (high glucose) (Invitrogen; Cat No. 11965-092) medium supplemented with 1X antibiotic/antimycotic and 10% FBS was added, which was then centrifugated at 1000 rpm at 4°C for 5 hours. Next, after removal of the supernatant, 40 mL of a D-MEM (high glucose) medium supplemented with 1X antibiotic/antimycotic and 10% FBS was added, which was then centrifugated again at 1000 rpm at 4°C for 5 hours. Furthermore, after removal of the supernatant, 10 mL of a D-MEM (high glucose) (Invitrogen; Cat No. 11965-092) medium supplemented with 1X antibiotic/antimycotic and 10% FBS was added, which was then seeded on a collagen-coated dish (60 mm) (Iwaki; Cat No. 11-018-004).

After 24 hours, the medium was removed, 5 mL of a retroviral vector solution containing the three genes (POU5F1, KLF4, and SOX2 at a ratio of 4:2:1 in that order) was added, and infected at 37°C for about 24 hours. The viral supernatant was removed, and mitomycin treated mouse embryonic fibroblasts (DS Pharma Biomedical; Cat No. R-PMEF-CF) was suspended at a density of 5.0×10⁴ cells/cm² in 5 mL of a D-MEM (high glucose) medium supplemented with 1X antibiotic/antimycotic (Invitrogen; Cat No. 15240-062) and 10% FBS; thereafter, which was then seeded on a collagen-coated dish (60 mm) on which the transfected cells derived from the non-cancer tissues of the stomach cancer patient had been cultured, and co-culturing was performed.

From then on, a MEF conditioned ES medium was repeatedly replaced every three days, and 31 days after the introduction of the three genes, mTeSR1 was replaced everyday. Fourty-six days after the gene transfection, one clone of a cell colony (NGC1-2) was picked up and subjected to passage culture on mitomycin treated mouse embryonic fibroblasts in a gelatin-coated 24-well plate. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² the day before the pickup of induced hepatic stem cells.

Listed below are the passage numbers (p) of induced human hepatic stem cells derived from non-cancer tissues of a stomach cancer patient, and the days when they were subjected to passage culture and lysed in a buffer for an RNA collection kit.

### <Induced human hepatic stem cells derived from non-cancer tissues of a stomach cancer patient>

### NGC1-2

Day 52: 24-well (p1) → 6-well (p2)
Day 58: 6-well (p2) → 10 cm (p3)
Day 65: Passage, stock and treatment with a buffer RLT (solution for lysing cells before RNA purification) (p4)

### EXAMPLE 5

### 5. Preparation of induced human hepatic stem cells from cells derived from cancer tissues of a colon cancer patient

To cells derived from fresh cancer tissues of a sigmoid colon cancer patient, a retroviral vector solution containing the three genes (POU5F1, KLF4, and SOX2 at a ratio of 4:2:1 in that order) was added for gene transfection to thereby prepare induced human hepatic stem cells. The details of the procedure are as described below.

Part of colon cancer tissues obtained during operation (from a 55-year-old Japanese male patient with sigmoid colon cancer) was washed with Hank's balanced salt solution (Phenol Red-free) (Invitrogen; Cat No. 14175-095) and minced with scissors into pieces of about 1 mm². The pieces were washed with Hank's balanced salt solution (Phenol Red-free) until a transparent supernatant was obtained. Thereafter, the supernatant was removed, 5 mL of a mixture of 0.01% collagenase (Wako Pure Chemical; Cat No. 034-10533) and 1X antibiotic/antimycotic (Invitrogen; Cat No. 15240-062) was added to the tissue precipitate, and stirring was performed at 37°C for 60 minutes with a shaker.

After the tissue precipitate was confirmed to have been fully digested, 35 mL of a D-MEM (high glucose) (Invitrogen; Cat No. 11965-092) medium supplemented with 1X antibiotic/antimycotic and 10% FBS was added, which was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, which was then centrifuged again at 1000 rpm at 4°C for 5 minutes. After removal of the supernatant, 10 mL of a D-MEM (high glucose) medium supplemented with 1X antibiotic/antimycotic and 10% FBS was added, which was then seeded on a collagen-coated dish (100 mm) (Iwaki; Cat No. 11-018-006).

After 24 hours, the medium was removed, and 10 mL of a retroviral vector solution containing the three genes was added. Five hours thereafter, 5 mL of a Luc-IRES-GFP retroviral vector solution was added, and infected at 37°C for about 24 hours. The viral supernatant was removed, and mitomycin treated MEFs (DS Pharma Biomedical; Cat No. R-PMEF-CF) was suspended at a density of 5.0×10⁴ cells/cm² in 10 mL of a D-MEM (high glucose) (Invitrogen; Cat No. 15240-092) medium supplemented with 1X antibiotic/antimycotic (Invitrogen; Cat No. 15240-062) and 10% FBS; thereafter, which are seeded on a collagen-coated dish (60 mm) on which the transfected cells derived from the cancer tissues of the colon cancer patient had been cultured, and co-culturing was performed.

Thereafter, a MEF conditioned ES medium was repeatedly replaced every three days, and from 22 days after the gene transfection, mTeSR1 was replaced everyday. Thirty-one days after the gene transfection, one clone of a cell colony (CC1-4) was picked up and subjected to passage culture on mitomycin treated mouse embryonic fibroblasts (DS Pharma Biomedical; Cat No. R-PMEF-CF) in a gelatin-coated 24-well plate. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² the day before the pickup of induced hepatic stem cells.

Listed below are the passage numbers (p) of induced human hepatic stem cells derived from cancer tissues of a colon cancer patient, and the days when they were subjected to passage culture and lysed in a buffer for an RNA collection kit.

### <Induced human hepatic stem cells derived from cancer tissues of a colon cancer patient>

### CC1-4

Day 40: 24-well (p1) → 6-well (p2)
Day 45: 6-well (p2) → 10 cm (p3)
Day 51: Passage and stock (p4)
Day 54: Passage and stock (p5)
Day 59: Passage and stock (p5)
Day 63: Treatment with a buffer RLT (solution for lysing cells before RNA purification) (p5)

### EXAMPLE 6

### 6. Preparation of induced human hepatic stem cells from cells derived from adult skin tissues

Induced human hepatic stem cells were prepared from cells derived from adult skin tissues (product name: normal adult human skin fibroblasts; primary culture; Lonza; Lot No. 76582).

One vial of cryopreserved normal adult human skin fibroblasts (primary culture; Lonza; Lot No. 76582) was thawed in a water bath at 37°C and suspended in a D-MEM (high glucose) (Invitrogen; Cat No. 11965-092) medium supplemented with 1X antibiotic/antimycotic (Invitrogen; Cat No. 15240-062) and 10% FBS to thereby obtain 10 mL of a cell suspension. Then, the obtained cell suspension was centrifuged at 1000 rpm at 4°C for 5 minutes to remove the supernatant, and thereafter the remaining cells were resuspended in 20 mL of FGM-2 BulletKit. The cell suspension was added at a volume of 10 mL per well onto a 100 mm dish (Nunc; Cat No. 172958) whose well bottoms had been coated with matrigel (Becton, Dickinson) at a concentration of 20 µg/cm² for at least 30 minutes, whereby cells were seeded.

After about 24 hours, the medium was removed, and 10 mL of a retroviral vector solution containing the three genes was added, and infected at 37°C for 24 hours. The viral supernatant was removed, and 10 mL of a MEF conditioned ES medium was added. Thereafter, a MEF conditioned ES medium was repeatedly replaced every three days, and from 18 days after the gene transfection, mTeSR1 (STEMCELL Technologies) was replaced everyday. For 6 days from 28 days after the gene transfection, a MEF conditioned ES medium was replaced everyday. From 34 days after the gene transfection, mTeSR1 was further replaced everyday. Thirty-nine days after the gene transfection, one clone of a cell colony (AFB1-1) was picked up and subjected to passage culture on mitomycin treated mouse embryonic fibroblasts (DS Pharma Biomedical; Cat No. R-PMEF-CF) in a gelatin-coated 24-well plate. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² the day before the pickup of induced hepatic stem cells.

Listed below are the passage numbers (p) of induced human hepatic stem cells derived from adult skin tissues, and the days when they were subjected to passage culture and lysed in a buffer for an RNA collection kit.

### <Induced human hepatic stem cells derived from adult skin tissues>

### AFB1-1

Day 50: 24-well (p1) to 6-well (p2)
Day 54: 6-well (p2) to 10 cm (p3)
Day 59: Passage (p4)
Day 63: Passage (p5)
Day 67: Passage, stock and treatment with a buffer RLT (solution for lysing cells before RNA purification) (p5)

The passage culture performed in Examples 2-6 described above was as described below.

After removing the medium from the cultured cells and washing the cells with PBS (-), a dissociation solution was added. After standing at 37°C for 5 minutes, the dissociation solution was removed, 20 mL of a D-MEM (high glucose) (Invitrogen; Cat No. 11965-092) medium supplemented with 1X antibiotic/antimycotic (Invitrogen; Cat No. 15240-062) and 10% FBS (Invitrogen; Cat No. 26140-079) was added, which was then centrifuged at 1000 rpm at 4°C for 5 minutes. Next, after removal of the supernatant, 1X antibiotic/antimycotic (Invitrogen; Cat No. 15240-062), mTeSR, and 10 µM Y-27632 were added, and the cell suspension was seeded on the gelatin-coated 100 mm dish where MEF had been seeded at 1.0×10⁶ cells/dish.

The following two types of dissociation solutions were used for passage culture:
(1) 0.25% trypsin/1 mM EDTA solution (Invitrogen; Cat No. 25200-056); and
(2) a mixture containing:
   10 mL of 10 mg/mL Collagenase (Invitrogen; Cat No. 17104-019),
   1 mL of a 100 mM calcium chloride solution,
   59 mL of PBS,
   10 mL of a 2.5% trypsin solution (Invitrogen; Cat No. 15090-046), and
   20 mL of knockout serum replacement (Invitrogen; Cat No. 10828-028).

### EXAMPLE 7

### 7. Long-term culture of induced human pluripotent stem cells and induced human hepatic stem cells

Induced human hepatic stem cells (AFB1-1, NGC1-2) were subjected to passage culture for six months or longer. The exemplary medium used includes mTeSR1 (STEMCELL Technologies/VERITAS), a bFGF-supplemented medium for primate ES/iPS cells (ReproCELL), or bFGF-supplemented ReproStem (ReproCELL). In the case of using MEFs, a collagen- or gelatin-coated culture dish was used, and in the case of not using MEFs, a matrigel-coated culture dish was used. The results show that the addition of 0.05-0.5 µM of A-83-01 (TGF-β signaling inhibitor, TGF-β type I receptor ALK5 kinase, type I activin/nodal receptor ALK4 and ALK7 inhibitors) was useful for self-replication of induced human hepatic stem cells, and yielded highly satisfactory proliferation rate and morphology.

### EXAMPLE 8

### 8. Microarray-based quantitative analysis of hepatocyte marker genes and embryonic stem cell marker genes

Genome-wide gene expression (mRNA transcriptome) was analyzed using the Whole Human Genome Oligo DNA Microarray (4X44K) manufactured by Agilent Technologies.

### <Samples>

In Examples 2-6, the total RNAs and genomic DNAs of induced human hepatic stem cells (NFB1-3, GC1-2, NGC1-2, AFB1-1, and CC1-4) prepared in Examples 2-6 were extracted from the solutions that had been treated with a buffer RLT (solution for lysing cells before RNA purification), using the AllPrep DNA/RNA Mini Kit (50) (Qiagen; Cat No. 80204).

The total RNAs of induced human hepatic stem cells (NFB1-3, GC1-2, NGC1-2, AFB1-1, and CC1-4) were used as samples.

### <Testing procedure>

### (1) Quality check

The total RNAs were checked for their quality on the Agilent 2100 Bioanalyzer (Agilent Technologies) using the RNA LabChip (registered trademark of Agilent Technologies) Kit, and all of the RNA samples were found to be of good quality. The RNA concentrations and purities were also assessed using the NanoDrop ND-1000 (NanoDrop Technologies), and as a result, every sample was verified to contain the total RNA in an amount required for cRNA synthesis and at a high level of purity.

### (2) cRNA synthesis

According to the Agilent's protocol, double-stranded cRNA was synthesized from the total RNA (500 ng) of each sample using the Quick Amp Labeling kit (Agilent Technologies). From the prepared cDNA, cRNA was synthesized by *in vitro* transcription. During the synthesis, the cRNA was fluorescence-labeled by incorporating Cyanine-labeled CTP (Cyanine 3-CTP).

### (3) Hybridization

With the aid of the Gene Expression Hybridization Kit (Agilent Technologies), the labeled cRNA for hybridization was added to a hybridization buffer to perform hybridization for 17 hours on the Whole Human Genome Oligo DNA Microarray (4X44K) manufactured by Agilent Technologies. After washing, DNA microarray images were scanned with an Agilent microarray scanner, and the fluorescent signals at each spot were converted to numerical values using the Feature Extraction Software (v.9.5.3.1).

### <Results of quantitative gene analysis>

The presence or absence of expression was evaluated with the median value of the total gene expression profile (distribution of fluorescence values for respective probes) taken as 0. A probe that showed an expression value of more than 0 was regarded as the a probe that detected the expression of genes, was assumed to have given rise to the expression of genes, and was counted in the number of expression probes.

It should be noted that the analysis software used was GeneSpring GX 10.0 (Agilent Technologies, Inc.) and that normalization was performed using the 50th percentile method. The microarray data for human embryonic stem cells (hES_ES01) to be used as a control was downloaded from GEO.

### 1. Genes characteristically expressed in hepatocytes

Table 6 below lists the genes that are characteristically expressed in hepatocytes and which are expressed in the induced human hepatic stem cell of the present invention. Among the 156 expressed probes (144 genes) on the Whole Human Genome Oligo DNA Microarray (4X44K) manufactured by Agilent Technologies, which were characteristically expressed in hepatocytes, those expressed in induced human hepatic stem cells were counted, and their Probe names, GeneSymbols, and GeneBank Accession Nos. are listed in the respective tables.

**[Table 6]**

| ProbeName | GeneSymbol | Genbank Accession | ProbeName | GeneSymbol | Genbank Accession | ProbeName | GeneSymbol | Genbank Accession |
|---|---|---|---|---|---|---|---|---|
| A_23_P116898 | A2M | NM_000014 | A_24_P347431 | FOXA1 | NM_004496 | A_23_P205355 | SERPINA5 | NM_000624 |
| A_23_P252981 | ACE2 | NM_021804 | A_23_P91552 | FTCD | NM_206965 | A_23_P41390 | SH3TC1 | NM_018986 |
| A_24_P324783 | ACVRL1 | NM_000020 | A_23_P384761 | GATA4 | NM_002052 | A_23_P66739 | SLC13A5 | NM_177550 |
| A_24_P945113 | ACVRL1 | NM_000020 | A_23_P129064 | GATM | NM_001482 | A_23_P102391 | SLC40A1 | NM_014585 |
| A_32_P196263 | ADAMTS9 | NM_182920 | A_23_P52227 | GDF10 | NM_004962 | A_24_P242581 | SLC5A9 | NM_001011547 |
| A_23_P406341 | AFAPIL2 | NM_001001936 | A_23_P250444 | GJB1 | NM_000166 | A_23_P150788 | SLCO2B1 | NM_007256 |
| A_23_P58205 | AFP | NM_001134 | A_32_P19294 | GLT1D1 | NM_144669 | A_23_P91230 | SLPI | NM_003064 |
| A_23_P115261 | AGT | NM_000029 | A_32_P109029 | GPRC5C | NM_022036 | A_23_P113351 | SPARCL1 | MM_004684 NM_0004684 |
| A 23 P155514 | AHSG | NM_001622 | A 23_P253495 | GSTA3 | NM_000847 | A_32_P133072 | SPON1 | NM_006108 |
| A_23_P155509 | AHSG | MM_001622 | A_23_P69573 | GUCY1A3 | NM_000856 | A_3_P354705 | ST8SIA1 | NM_003034 |
| A_24_P372189 | AK027294 | AK027294 | A_24_P52697 | H19 | NR_002196 | A_23_P36345 | STARD10 | MM.006645 |
| A_32_P56661 | AK074614 | AK074614 | A_23_P47034 | HHEX | NM_002729 | A_23_P399265 | STMN2 | S82024 |
| A_32_P23525 | AK124281 | AK124281 | A_23_P202427 | HKDC1 | NM_025130 | A_23_P80974 | TDO2 | NM_005651 |
| 4_24_P766716 | AK126405 | AK126405 | A_23_P103588 | HMGCS2 | NM_005518 | A_73_P212500 | TF | NM_001063 |
| A_23_P257834 | ALB | NM_000477 | A_23_P206760 | HP | NM_005143 | A_23_P212508 | TF | NM_001063 |
| A_23_P83098 | ALDH1A1 | NM_000689 | A_23_P421493 | HPR | NM_020995 | A_23_P101013 | TMC6 | NM_007267 |
| A_32_P105549 | 4NXA8 | NM_001630 | A_23_P161998 | HPX | NM_000813 | A_23_P409093 | TMEM16D | NM_178826 |
| A_23_P337262 | APCDD1 | NM_153000 | A_23_P118065 | HSD17B2 | NM_002153 | A_32_P7015 | TSPAN15 | NM_012339 |
| A_23_P203191 | APOA1 | NM_000039 | A_23_P395438 | HTRA3 | NM_053044 | A_23_P130333 | TTR | NM_000371 |
| A_24_P302249 | APOA2 | NM_001643 | A_23_P10542 | HTRA3 | NM_053044 | A_23_P81888 | UBD | NM_006398 |
| A_23_P87036 | APOA4 | NM_000482 | A_23_P150609 | IGF2 | NM_001007139 | A_P212968 | UGT2B11 | NM_001073 |
| A_23_P78591 | APOB | NM_000384 | A_23_P15146 | IL32 | NM_001012931 | A_23_P136671 | UGT2B7 | NM_001074 |
| A_23_P259071 | AREG | NM_001657 | A_23_P153964 | INHBB | NM_002193 | A_23_P214408 | UNC93A | NM_018974 |
| A_23_P116902 | ART4 | NM_021071 | A_32_P217140 | ISX | NM_001008494 | A_24_P103434 | UNC93A | NM_018974 |
| A_23_P130113 | ASGR2 | NM_080912 | A_23_P501193 | KCNJ16 | NM_170741 | A_23_P34345 | VCAM1 | NM_001078 |
| A_23_P118894 | ATAD44 | NM_024320 | A_23_P56898 | KYNU | NM_003937 | A_23_P16866 | VIL1 | NM_007127 |
| A_24_P753592 | BC018589 | BC018589 | A_23_P201638 | LAMC2 | NM 005562 | A_23_P78099 | VTN | NM_000638 |
| A_23_P143331 | BMP2 | NM_001200 | A_23_P120902 | LGALS2 | NM_006498 | A_23_P106617 | WFDCt | NM_021197 |
| A_32_42224 | BX097190 | BX097190 | A_23_P32165 | LHX2 | NM_004789 | | | |
| A_23_P75790 | C11orf9 | NM_013278 | A_24_P178834 | LOC132205 | AK091178 | | | |
| A_23_P204937 | C13orf15 | NM_014059 | A_24_P463929 | LOC285733 | AK091900 | | | |
| A_24_P10137 | C13orf15 | NM 014059 | A_24_P845223 | M27126 | M27126 | | | |
| A_23_P88678 | C15orf27 | NM_152335 | A_24_P258219 | MAF | AF055376 | | | |
| A_23_P101407 | C3 | NM_000064 | A_23_P164057 | MFAP4 | NM_002404 | | | |
| A_23_P71855 | C5 | NM_001735 | A_23_P13094 | MMP10 | NM_002425 | | | |
| A_32_P213103 | CA414006 | CA414006 | A_23_P213171 | MTTP | NM_000253 | | | |
| A_23_P33723 | CD163 | NM_004244 | A_23_P102364 | NGEF | NM_019850 | | | |
| A_24_P11208 | CD1D | NM_001766 | A_23_P389897 | NGFR | NM_002507 | | | |
| A_23_P76654 | CDX2 | NM_001265 | A_24_P252364 | NRCAM | NM_005010 | | | |
| A_23_P151895 | CILP | NM_003613 | A_23_P360797 | NTF3 | NM_002527 | | | |
| A_23_P105461 | CMKLR1 | NM_004072 | A_24_P220485 | OLFML2A | NM_182487 | | | |
| A_23_P217379 | COL4A6 | NM_033641 | A_32_P61684 | PAG1 | NM_018440 | | | |
| A_23_P120125 | COLEC11 | NM_199235 | A_23_P347070 | PAG1 | NM_018440 | | | |
| A_24_P388322 | COLEC11 | NM_199235 | A_23_P151907 | PCSK6 | NM_002570 | | | |
| A 23 P213745 | CXCL14 | NM_004887 | A_24_P243748 | PDK4 | NM_002812 | | | |
| A_23_P102000 | CXCR4 | NM_001008540 | A_23_P52121 | PDZK1 | NM_002614 | | | |
| A_23_P131676 | CXCR7 | NM_020311 | A_23_P388150 | PLA2G12B | NM_032562 | | | |
| A_23_P32577 | DACH1 | NM_080759 | A_32_P206123 | PLG | NM_000301 | | | |
| A_23_P257583 | DENND2A | NM_015689 | A_23_P30693 | PLG | NM_000301 | | | |
| A_23_P105923 | DIO3 | NM_001362 | A_23_P160286 | PRG4 | NM_005807 | | | |
| A_24_P236251 | DLK1 | NM_003836 | A_32_P157391 | PSMAL | NM_153696 | | | |
| A_23_P139704 | DUSP6 | NM_001946 | A_23_P146554 | PTGDS | NM_000954 | | | |
| A_23_P139687 | ERP27 | NM_152321 | A_23_P167030 | PTHR1 | NM_000316 | | | |
| A_23_P150379 | EVA1 | MM_144765 | A_23_P118392 | RASD1 | NM_016084 | | | |
| A_23_P205177 | F10 | NM_000501 | A_23_P75283 | RBP4 | NM_006744 | | | |
| A_23_P94879 | F2 | NM_000506 | A_23_P3934 | RNF43 | NM_017763 | | | |
| A_23_P79562 | FABP1 | NM_001443 | A_23_P88849 | RRAD | NM_004165 | | | |
| A_23_P375372 | FGA | NM_021871 | A_24_P262127 | RRAD | NM_004165 | | | |
| A_23_P44274 | FGA | NM_000508 | A_23_P124619 | S100A14 | NM_020672 | | | |
| A_23_P136125 | FGB | NM_005141 | A_23_P121926 | SEPP1 | MM.005410 | | | |
| A_23_P148088 | FGG | NM_000509 | A_24_P145629 | SERINC2 | NM_178865 | | | |
| A_23_P166109 | FLRT3 | NM_198391 | A_23_P218111 | SERPINA1 | NM_001002236 | | | |
| A_23_P114863 | FMOD | NM_002023 | A_23_P2920 | SERPINA3 | NM_001085 | | | |
| A_23_P37127 | FOXA1 | NM_004496 | A_24_P321766 | SERPINA5 | NM_000624 | | | |

Table 7 below lists the genes expressed in induced human hepatic stem cells (GC1-2) that were derived from cancer tissues of a stomach cancer patient and which were induced in Example 3.
Induced human hepatic stem cells derived from cancer tissues of a stomach cancer patient: GC1-2
The number of expressed probes characteristic of hepatocytes was 138.

**[Table 7]**

| ProbeName | GeneSymbol | GenbankAccession | ProbeName | GeneSymbol | GenbankAccession | ProbeName | GeneSymbol | GenbankAccession |
|---|---|---|---|---|---|---|---|---|
| A_23_P116898 | A2M | NM_000014 | A_24_P238251 | DLK1 | NM_003836 | A_23_P102364 | NGEF | NM_019850 |
| A_23_P252981 | ACE2 | NM_021804 | A_23_P139704 | DUSP6 | NM_001946 | A_23_P389897 | NGFR | NM_002507 |
| A_24_P324783 | ACVRL1 | NM_000020 | A_23_P139687 | ERP27 | NM_152321 | A_23_P380797 | NTF3 | NM_002527 |
| A_24_P945113 | ACVRL1 | NM_000020 | A_23_P205177 | F10 | NM 000504 | A_24_P220485 | OLFML2A | NM_182487 |
| A_32_P196263 | ADAMTS9 | NM_182920 | A_23_P94879 | F2 | NM_000506 | A_32_P61684 | PAG1 | NM_018440 |
| A_23_P408341 | AFAP1L2 | NM_001001936 | A_23_P79562 | FABP1 | NM_001443 | A_23_P347070 | PAG1 | NM_018440 |
| A_23_P58205 | AFP | NM_001134 | A_23_P375372 | FGA | NM_021871 | A_23_P52121 | PDZK1 | NM_002914 |
| A_23_P115261 | AGT | NM_000029 | A_23_P44274 | FGA | NM_000508 | A_23_P388150 | PLA2G12B | NM_032562 |
| A_23_P155514 | AHSG | NM_001622 | A_23_P138125 | FOB | NM_005141 | A_32_P208123 | PLG | NM_000301 |
| A_23_P155509 | AHSG | NM_001622 | A_23_P148088 | FGG | NM_000509 | A_23_P30693 | PLG | NM_000301 |
| A_24_P372189 | AK027294 | AK027294 | A_23_P166109 | FLRT3 | NM_198391 | A_23_P160286 | PRG4 | NM_005807 |
| A_32_P58661 | AK074614 | AK074614 | A_23_P114883 | FMOD | NM_002023 | A_23_P146554 | PTGDS | NM_000954 |
| A_32_P23525 | AK124281 | AK124281 | A_23_P37127 | FOXA1 | NM 004496 | A_23_P167030 | PTHR1 | NM_000316 |
| A_24_P766716 | AK126405 | AK126405 | A_24_P347431 | FOXA1 | NM_004496 | A_23_P118392 | RASD1 | NM_016084 |
| A_23_P257834 | ALB | NM_000477 | A_23_P384761 | GATA4 | NM_002052 | A_23_P75283 | RBP4 | NM_006744 |
| A_23_P83098 | ALDH1A1 | NM_000689 | A_23_P129064 | GATM | NM_001482 | A_23_P3934 | RNF43 | NM_017763 |
| A_32_P105549 | ANXA8 | NM_001630 | A_23_P250444 | GJB1 | NM_000166 | A_23_P88849 | RRAD | NM_004165 |
| A_23_P337262 | APCDD1 | NM_153000 | A_32_P19294 | GLT1D1 | NM_144669 | A_24_P282127 | RRAD | MM_004165 |
| A_23_P203191 | APOA1 | NM_000039 | A_32_P109029 | GPRC5C | NM_022036 | A_23_P124819 | S100A14 | NM_020672 |
| A_24_P302249 | APOA2 | NM_001643 | A_23_P253495 | GSTA3 | NM_000847 | A_23_P121926 | SEPP1 | NM_005410 |
| A_23_P87036 | APOA4 | NM_000482 | A_23_P69573 | GUCY1A3 | NM_000856 | A_24_P145629 | SERINC2 | NM_178865 |
| A_23_P79591 | APOB | NM_000384 | A_24_P52697 | H19 | NR_002196 | A_23_P218111 | SERPINA1 | NM_001002236 |
| A_23_P259071 | AREG | NM_001657 | A_23_P47034 | HHEX | NM_002729 | A_24_P321766 | SERPINA5 | NM_000624 |
| A_23_P116902 | ART4 | NM_021071 | A_23_P103588 | HMGCS2 | NM_005518 | A_23_P205355 | SERPINA5 | NM_000624 |
| A_23_P130113 | ASGR2 | NM_080912 | A_23_P206760 | HP | NM_005143 | A_23_P41390 | SH3TC1 | NM_018988 |
| A_24_P753592 | BC018589 | BC018589 | A_23_P421493 | HPR | NM_020995 | A_23_P102391 | SLC40A1 | NM_014585 |
| A_23_P143331 | BMP2 | NM_001200 | A_23_P161998 | HPX | NM_000613 | A_24_P242581 | SLC5A9 | NM_001011517 |
| A_32_P42224 | BX097190 | BX097190 | A_23_P118065 | HSD17B2 | NM_002153 | A_32_P133072 | SPON1 | NM_006108 |
| A_23_P75790 | C11orf9 | NM_013279 | A 23 P395438 | HTRA3 | NM_053044 | A_23_P354705 | ST8SIA1 | NM_003034 |
| A_23_P204937 | C13orf15 | NM_014059 | A_23_P10542 | HTRA3 | NM_053044 | A_23_P36345 | STARD10 | NM_006645 |
| A_24_P10137 | C13orf15 | NM_014059 | A_23_P150609 | IGF2 | NM_001007139 | A_23_P399265 | STMN2 | S82024 |
| A_23_P88678 | C15orf27 | NM_152335 | A_23_P15146 | IL32 | NM_0012631 | A_23_P212500 | TF | NM_001063 |
| A_23_P101407 | C3 | NM_000064 | A_23_P153964 | INHBB | NM 002193 | A_23_P212508 | TF | NM_001063 |
| A_23_P71855 | C5 | NM_001735 | A_32_P217140 | ISX | NM_001008494 | A_23_P101013 | TMC6 | NM_007267 |
| A_32_P213103 | CA414006 | CA414006 | A_23_P501193 | KCNJ16 | NM_170741 | A_23_P409093 | TMEM16D | NM_178826 |
| A_23_P33723 | CD163 | NM_004244 | A_23_P56898 | KYNU | NM_003937 | 4_32_P7015 | TSPAN15 | NM_012339 |
| A_23_P151895 | CILP | NM_003613 | A_23_P201636 | LAMC2 | NM_005562 | A_23_P130333 | TTR | NM_000371 |
| A_23_P217379 | COL4A6 | NM_033641 | A_23_P120902 | LGALS2 | NM_006498 | A_23_P81898 | UBD | NM_006398 |
| A_23_P120125 | COLEC11 | NM_199235 | A_23_P32165 | LHX2 | NM_004789 | A_23_P212968 | UGT2B11 | NM_001073 |
| A_24_P388322 | COLEC11 | NM_199235 | A_24_P178834 | LOC132205 | AK091178 | A_23_P138871 | UGT2B7 | NM_001074 |
| A_23_P213745 | CXCL14 | NM_004887 | A_24_P463929 | LOC285733 | AK091900 | A_23_P214408 | UNC93A | NM_018974 |
| A_23_P102000 | CXCR4 | NM_001008540 | A_24_P845223 | M27126 | M27126 | A_24_P103434 | UNC93A | NM_018974 |
| A_23_P131676 | CXCR7 | NM_020311 | A_24_P256219 | MAF | AF055376 | A_23_P34345 | VCAM1 | NM_001078 |
| A_23_P32577 | DACH1 | NM_080759 | A_23_P164057 | MFAP4 | NM_002404 | A_23_P16866 | VIL1 | NM_007127 |
| A_23_P257583 | DENND2A | NM_015689 | A_23_P13094 | MMP10 | NM_002425 | A_23_P78099 | VTN | NM_000838 |
| A_23_P105923 | DIO3 | NM_001362 | A_23_P213171 | MTTP | NM_000253 | A_23_P108817 | MFDC1 | NM_021197 |

Table 8 below lists the genes expressed in induced human hepatic stem cells (AFB1-1) that were derived from adult skin tissues and which were induced in Example 6. Induced human hepatic stem cells derived from adult skin tissues: AFB1-1 The number of expressed probes characteristic of hepatocytes was 133.

**[Table 8]**

| ProbaName | GeneSymbol | GenbankAccession | ProbeName | GeneSymbol | GenbankAccession | ProbeName | GeneSymbol | GenbenkAccession |
|---|---|---|---|---|---|---|---|---|
| A_23_P118898 | A2M | NM_000014 | A_23_P150379 | EVA1 | MM_144765 | A_23_P360797 | NTF3 | NM_002527 |
| A_23_P252981 | ACE2 | NM_021804 | A_13_P205177 | F10 | NM_000504 | A_24_P220485 | OLFML2A | NM_182487 |
| A_24_P945113 | ACVRL1 | NM_000020 | A_23_P94879 | F2 | NM_000506 | A_32_P81684 | PAG1 | NM_018440 |
| A_32_P198283 | ADAMTS9 | NM_182920 | A_23_P79562 | FABP1 | NM_001443 | A_23_P347070 | PAG1 | NM_018440 |
| A_23_P406341 | AFAP1L2 | NM_001001936 | A 23_P375372 | FGA | NM_021871 | A_23_P151907 | PCSK6 | NM_002570 |
| A_23_P58205 | AFP | NM_001134 | A_23_P44274 | FGA | NM_000508 | A_24_P243749 | PDK4 | NM_002612 |
| A_23_P115261 | AGT | NM_000029 | A_23_P136125 | FGB | NM_005141 | A_21_P52121 | PDZK1 | NM_002614 |
| A_23_P155514 | AHSG | NM_001622 | A_23_P148088 | FGG | NM_000509 | A_32_P206123 | PLG | NM_000301 |
| A_23_P155509 | AHSG | NM_001622 | A_23_P166109 | FLRT3 | NM_198391 | A_23_P30693 | PLG | NM_000301 |
| A_24_P372189 | AK027294 | AK027294 | A_23_PH4893 | FMOD | NM_002023 | A_23_P160286 | PRG4 | NM_005807 |
| A_32_P58661 | AK074614 | AK074614 | A_24_P347431 | FOXA1 | NM_004496 | A_23_N148554 | PTGDS | NM_000954 |
| 4_32_P23525 | AK124281 | AK124281 | A_23_P91552 | FTCD | NM_206965 | A_23_P187030 | PHR1 | NM_000316 |
| A_24_P786716 | AK128405 | AK126405 | A_23_P384761 | GATA4 | NM_002052 | A_23_P119392 | RASD1 | NM_016064 |
| A_23_P257834 | ALB | NM_000477 | A_23_P129064 | 3ATM | NM_001482 | A_23_P75283 | RBP4 | NM_008744 |
| A_23_P83098 | ALDH1A1 | NM_000689 | A_23_P52227 | GDF10 | NM_004982 | A_23_P3934 | RNF43 | NM_017763 |
| A_32_P105549 | ANXA8 | NM_001630 | A_23_P250444 | GJB1 | NM_000188 | A_23_P88849 | RRAD | NM_004165 |
| A_23_P337262 | APCDD1 | NM_153000 | A_32_P19294 | GLT1D1 | NM_144669 | A_24_P262127 | RRAD | NM_004165 |
| A_23_P203191 | APOA1 | NM_000039 | A_32_P109029 | GPRC5C | NM_022036 | A_23_P124619 | S100A14 | NM_020672 |
| A_24_P302249 | APOA2 | MM_001643 | A_23_P253495 | GSTA3 | NM_000847 | A_23_P121926 | SEPP1 | NM_005410 |
| A_23_P87038 | APOA4 | NM_000482 | A_23_P69573 | GUCY1A3 | NM_000856 | A_24_P145629 | SERINC2 | NM_178865 |
| A_23_P79591 | APOB | NM_000384 | A_24_P52697 | H19 | NR_002196 | A_23_P218111 | SERPINA1 | NM_001002236 |
| A_23_P259071 | AREG | NM_001657 | A_23_P47034 | HHEX | NM_002729 | A_23_P205355 | SERPINA5 | NM_000624 |
| A_23_P130113 | ASGR2 | NM_080912 | A_23_P202427 | HKDC1 | NM_025130 | A_23_P41390 | SH3TC1 | NM_018988 |
| A_24_P753592 | BC018589 | BC018589 | A_23_P103588 | HMGCS2 | NM_005518 | A_23_P66739 | SLC13A5 | NM_177550 |
| A_23_P143331 | BMP2 | NM_001200 | A_23_P206760 | HP | NM_005143 | A_23_P102391 | SLC40A1 | NM_014585 |
| A_32_P42224 | BX097190 | BX097190 | A_23_P161998 | HPX | NM_000613 | A_23_P150768 | SLCO2B1 | NM_007256 |
| A_23_P75790 | C11 | NM_013279 | A_23_P118065 | HSD17B2 | NM_002153 | A_23_P91230 | SLPI | NM_003064 |
| A_23_P204937 | C13orf15 | NM_014059 | A_23_P395438 | HTRA3 | NM_053044 | A_23_P113351 | SPARCL1 | NM_004684 |
| A_24_P10137 | C13orf15 | NM_014059 | A_23_P10542 | HTRA3 | NM_053044 | A_32_P133072 | SPON1 | NM_006108 |
| A_23_P88678 | C15orf27 | NM_152335 | A_23_P150609 | IGF2 | NM_001007139 | A_23_P38345 | STARD10 | NM_006645 |
| A_23_P101407 | C3 | NM_000064 | A_23_P15146 | IL32 | NM_001012631 | A_23_P399265 | STMN2 | S82024 |
| A_23_P71855 | C5 | NM_001735 | A_23_P153964 | INHBB | NM_002193 | A_23_P212500 | TF | NM_001063 |
| A_32_P213103 | CA414006 | CA414006 | A_23_P501193 | KCNJ16 | NM_170741 | A_23_P212508 | TF | NM_001063 |
| A_23_P151895 | CILP | NM_003613 | A_23_P58898 | KYNU | NM_003937 | A_23_P101013 | TMC6 | NM_007267 |
| A_23_P217379 | COL4A6 | NM_033641 | A_23_P201636 | LAMC2 | NM_005562 | A_32_P7015 | TSPAN15 | NM_012339 |
| A_23_P120125 | COLEC11 | NM_199235 | A_23_P120902 | LGALS2 | NM_006498 | A_23_P130333 | TTR | NM_000371 |
| A_24_P388322 | COLEC11 | NM_199235 | A_23_P32165 | LHX2 | NM_004789 | A_23_P81898 | UBD | NM_006398 |
| A_23_P213745 | CXCL14 | NM_004887 | A_24_P178834 | LOC132205 | AK091178 | A_23_P212968 | UGT2B11 | NM_001073 |
| A_23_P102000 | CXCR4 | NM_001008540 | A_24_P463929 | LOC285733 | AK091900 | A_23_P136671 | UGT2B7 | NM_001074 |
| A_23_P131676 | CXCR7 | NM_020311 | A_24_P845223 | M27126 | M27126 | A_23_P34345 | VCAM1 | NM_001078 |
| A_23_P257583 | DENND2A | NM_015689 | A_23_P164057 | MFAP4 | NM_002404 | A_22_P16866 | VIL1 | NM_007127 |
| A_23_P105923 | DIO3 | NM_001362 | A_23_P213171 | MTTP | NM_000253 | A_23_P78099 | VTN | NM_000638 |
| A_24_P236251 | DLK1 | NM_003836 | A_23_P102364 | NGEF | NM_019850 | A_23_P106617 | WFDC1 | NM_021197 |
| A_23_P139704 | DUSP6 | NM_001946 | A_23_P389897 | NGFR | NM_002507 | | | |
| A_23_P139687 | ERP27 | NM_152321 | A_24_P252364 | NRCAM | NM_005010 | | | |

Table 9 below lists the genes expressed in induced human hepatic stem cells (NGC1-2) that were derived from non-cancer tissues of a stomach cancer patient and which were induced in Example 4.
Induced human hepatic stem cells derived from non-cancer tissues of a stomach cancer patient: NGC1-2
The number of expressed probes characteristic of hepatocytes was 131.

**[Table 9]**

| ProbeName | GeneSymbol | GenbankAccession | ProbeName | GeneSymbol | GenbankAccession | ProbeName | GeneSymbol | GenbankAccession |
|---|---|---|---|---|---|---|---|---|
| A_23_P116898 | A2M | NM_000014 | A_23_P1059223 | DIO3 | NM_001362 | A_23_P213171 | MTTP | NM_000253 |
| A_23_P252981 | ACE2 | NM_021804 | A_24_P236251 | CLK1 | NM_003836 | A_23_P102384 | NGEF | NM_019850 |
| A_24_P945113 | ACVRL1 | NM_000020 | A_23_P139704 | DUSP6 | NM_001946 | A_23_P389897 | NGFR | NM_002507 |
| A_32_P196283 | ADAMTS9 | NM_182920 | A_23_P139687 | ERP27 | NM_152321 | A_23_P380797 | NTF3 | NM_002527 |
| A_23_P408341 | AFAP1L2 | NM_001001938 | A_23_P205177 | F10 | NM_000504 | A_24_P220485 | OLFML2A | NM_182487 |
| A_23_P58205 | AFP | NM_001134 | A_23_P94879 | F2 | NM 000506 | A_32_P61684 | PAG1 | NM_018440 |
| A_23_P115261 | AGT | NM_000029 | A_23_P79562 | FABP1 | NM_001443 | A_23_P347070 | PAG1 | NM_018440 |
| A_23_P155514 | AHSG | NM_001822 | A_23_P375372 | FGA | NM_021871 | A_23_P52121 | PDZK1 | NM_002614 |
| A_23_P155509 | AHSG | NM_001622 | A_23_P44274 | FGA | NM_000508 | A_32_P206123 | PLG | NM_000301 |
| A_24_P372189 | AK027294 | AK027294 | A_23_P136125 | FGB | NM_005141 | A_23_P30893 | PLG | NM_000301 |
| A_32_P56661 | AK074614 | AK074614 | A_23_P148088 | FGG | NM_000509 | A_23_P160286 | PRG4 | NM_005807 |
| A_32_P23525 | AK124281 | AK124281 | A_23_P166109 | FLRT3 | NM_198391 | A_23_P146554 | PTGDS | NM_000954 |
| A_24_P706716 | AK126405 | AK126405 | A_23_P114883 | FMOD | NM_002023 | A_23_P167030 | PTHR1 | NM_000316 |
| A_23_P257834 | ALB | NM_000477 | A_23_P37127 | FOXA1 | NM_004496 | A_23_P118392 | RASD1 | NM_016084 |
| A_23_P83098 | ALDH1A1 | NM_000889 | A_24_P347431 | FOXA1 | NM_004496 | A_23_P75283 | RBP4 | NM_006744 |
| A_32_P105549 | ANXA8 | NM_001830 | A_23_P91552 | FTCD | NM_06965 | A_23_P3934 | RNF43 | NM_017763 |
| A_23_P337262 | APCDD1 | NM_153000 | A_23_P384761 | GATA4 | NM_002052 | A_23_P88849 | RRAD | NM_004165 |
| A_23_P203191 | APOA1 | NM_000039 | A_23_P129064 | GATM | NM_001482 | A_24_P262127 | RRAD | NM_004165 |
| A_24_P302249 | APOA2 | NM_001643 | A_23_P250444 | GJB1 | NM_000166 | A_23_P124619 | S100A14 | NM_020672 |
| 4_23_P87036 | APOA4 | NM_000482 | A_32_P19294 | GLT1D1 | NM_144669 | A_23_P121926 | SEPP1 | NM_005410 |
| A_23_P79591 | APOB | NM_000384 | A_32_P109029 | GPRC5C | NM_022038 | A_24_P145629 | SERINC2 | NM_178885 |
| A_23_P259071 | AREG | NM_001657 | A_23_P253495 | GSTA3 | NM_000847 | A_23_P218111 | SERPINA1 | NM_001002236 |
| A_23_P116902 | ART4 | NM_021071 | A_23_P69573 | GUCY1A3 | NM_000856 | A_23_P205355 | SERPINA5 | NM_000624 |
| A_23_P130113 | ASGR2 | NM_080912 | A_24_P52697 | H19 | NR_002196 | A_23_P66739 | SLC13A5 | NM_177550 |
| A_23_P118894 | ATAD4 | NM_024320 | A_23_P47034 | HHEX | NM_002729 | A_23_P102391 | SLC40A1 | NM_014585 |
| A_24_P753592 | BC018589 | BC018589 | A_23_P103588 | HMGCS2 | NM_005518 | A_23_P91230 | SLPI | NM_003064 |
| A_23_P143331 | BMP2 | NM_001200 | A_23_P209760 | HP | NM_005143 | A_32_P133072 | SPON1 | NM_006108 |
| A_32_P42224 | BX097190 | BX097190 | A_23_P161998 | HPX | NM_00613 | A_23_P36345 | STARD10 | NM_006645 |
| A_23_P75790 | C11orf9 | NM_013279 | A_23_P118065 | HSD17B2 | NM_002153 | A_23_P399265 | STMN2 | S82024 |
| A_23_P204937 | C13orf15 | NM_014059 | A_23_P395438 | HTRA3 | NM_053044 | A_23_P80974 | TDO2 | NM_005651 |
| A_24_P10137 | C13orf15 | NM_014059 | A_23_P10542 | HTRA3 | NM_053044 | A_23_P212500 | TF | NM_001063 |
| A_23_P88678 | C15orf27 | NM_152335 | A_23_P150609 | IGF2 | NM_001007139 | A_23_P212508 | TF | NM_001063 |
| A_23_P101407 | C3 | NM_000064 | A_23_P15146 | IL32 | NM_001012631 | A_23_P101013 | TMC6 | NM_007267 |
| A_23_P71855 | C5 | NM_001735 | A_23_P153964 | INHBB | NM_002193 | A_23_P409093 | TMEM16D | NM_178826 |
| A_32_P213103 | CA414006 | C414006 | A_32_P217140 | ISX | NM_001008494 | A_32_P7015 | TSPAN15 | NM_012339 |
| A_23_P151895 | CILP | NM_003613 | A_23_P501193 | KCNJ16 | NM_170741 | A_23_P130333 | TTR | NM_000371 |
| A_23_P217379 | COL4A6 | NM_033641 | A_23_P56898 | KYNU | NM_003937 | A_23_P81898 | UBD | NM_006398 |
| A_23_P120125 | COLEC11 | NM_199235 | A_23_P201636 | LAMC2 | NM_005562 | A_23_P212968 | UGT2B11 | NM_001073 |
| A_24_P388322 | COLEC11 | NM_199235 | A_23_P120902 | LGALS2 | NM_008498 | A_23_P136671 | UGT2B7 | NM_001074 |
| A_23_P213745 | CXCL14 | NM_006887 | A_23_P32165 | LHX2 | NM_004789 | A_23_P214408 | UNC93A | NM_018974 |
| A_23_P102000 | CXCR4 | NM_001008540 | A_24_P178834 | LOC132205 | AK091178 | A_23_P16866 | VIL1 | NM_007127 |
| A_23_P131676 | CXCR7 | NM_020311 | A_24_P463929 | LOC285733 | AK091900 | A_23_P78099 | VTN | NM_000638 |
| A_23_P32577 | DACH1 | NM_080759 | A_24_PB845223 | M27126 | M27126 | A_23_P108817 | WFDC1 | NM_021197 |
| A_23_P257583 | DENND2A | NM_015689 | A_2 _P184057 | MFAP4 | NM_002404 | | | |

Table 10 below lists the genes expressed in induced human hepatic stem cells (NFB1-3) that were derived from neonatal skin tissues and which were induced in Example 2. Induced human hepatic stem cells derived from neonatal skin tissues: NFB1-3
The number of expressed probes characteristic of hepatocytes was 96.

**[Table 10]**

| ProbeName | GeneSymbol | GenbankAccession | ProbeName | GeneSymbol | GenbankAccession |
|---|---|---|---|---|---|
| A_23_P116898 | A2M | NM_000014 | A_23_P69573 | GUCY1A3 | NM_000856 |
| A_23_P252981 | ACE2 | NM_021804 | A_24_P52697 | H19 | NR_002196 |
| A_24_P945113 | ACVRL1 | NM_000020 | A_23_P206760 | HP | NM_005143 |
| A_32_P196263 | ADAMTS9 | NM_182920 | A_23_P395438 | HTRA3 | NM_053044 |
| A_23_P406341 | AFAP1L2 | NM_001001936 | A_23_P150609 | IGF2 | NM_001007139 |
| A_23_P58205 | AFP | NM_001134 | A_23_P15146 | IL32 | NM_001012631 |
| A_23_P155509 | AHSG | NM_001622 | A_23_P501193 | KCNJ16 | NM_170741 |
| A_23_P83098 | ALDH1A1 | NM_000689 | A_23_P56898 | KYNU | NM_003937 |
| A_32_P105549 | ANXA8 | NM_001630 | A_23_P201636 | LAMC2 | NM_005562 |
| A_23_P337262 | APCDD1 | NM_153000 | A_23_P120902 | LGALS2 | NM_006498 |
| A_23_P203191 | APOA1 | NM_000039 | A_23_P32165 | LHX2 | NM_004789 |
| A_24_P302249 | APOA2 | NM_001643 | A_24_P178834 | LOC132205 | AK091178 |
| A_23_P87036 | APOA4 | NM 000482 | A_23_P164057 | MFAP4 | NM_002404 |
| A_23_P116902 | ART4 | NM_021071 | A_23_P13094 | MMP10 | NM_002425 |
| A_23_P130113 | ASGR2 | NM_080912 | A_23_P213171 | MTTP | NM_000253 |
| A_24_P753592 | BC018589 | BC018589 | A_23_P102364 | NGEF | NM_019850 |
| A_23_P143331 | BMP2 | NM_001200 | A_23_P389897 | NGFR | NM_002507 |
| A_32_P42224 | BX097190 | BX097190 | A_24_P252364 | NRCAM | NM_005010 |
| A_23_P75790 | C11orf9 | NM_013279 | A_23_P360797 | NTF3 | NM_002527 |
| A_23_P204937 | C13orf15 | NM_014059 | A_24_P220485 | OLFML2A | NM_182487 |
| A_24_P10137 | C13orf15 | MM_014059 | A_32_P61684 | PAG1 | NM_018440 |
| A_32_P213103 | CA414006 | CA414006 | A_23_P347070 | PAG1 | NM_018440 |
| A_23_P76654 | CDX2 | NM_001265 | A_23_P52121 | PDZK1 | NM_002614 |
| A_23_P217379 | COL4A6 | NM_033641 | A_23_P388150 | PLA2G12B | NM_032562 |
| A_23_P120125 | COLEC11 | NM_199235 | A_23_P146554 | PTGDS | NM_000954 |
| A_23_P213745 | CXCL14 | NM_004887 | A_23_P167030 | PTHR1 | NM_000316 |
| A_23_P102000 | CXCR4 | NM_001008540 | A_23_P118392 | RASD1 | NM_016084 |
| A_23_P131676 | CXCR7 | NM_020311 | A_23_P75283 | RBP4 | NM_006744 |
| A_23_P32577 | DACH1 | NM_080759 | A_23_P3934 | RNF43 | NM_017763 |
| A_23_P257583 | DENND2A | NM_015689 | A_23_P88849 | RRAD | NM_004165 |
| A_23_P105923 | DIO3 | NM_001362 | A_23_P124619 | S100A14 | NM_020672 |
| A 24_P236251 | DLK1 | NM_003836 | A_23_P121926 | SEPP1 | NM_005410 |
| A_23_P139704 | DUSP6 | NM_001946 | A_24_P321766 | SERPINA5 | NM_000624 |
| A_23_P205177 | F10 | NM_000504 | A_23_P205355 | SERPINA5 | NM_000624 |
| A_23_P94879 | F2 | NM_000506 | A_23_P102391 | SLC40A1 | NM_014585 |
| A_23_P79562 | FABP1 | NM_001443 | A_23_P91230 | SLPI | NM_003064 |
| A_23_P136125 | FGB | NM_005141 | A_32_P133072 | SPON1 | NM_006108 |
| A_23_P148088 | FGG | NM_000509 | A_23_P354705 | ST8SIA1 | NM_003034 |
| A_23_P166109 | FLRT3 | NM_198391 | A_23_P36345 | STARD10 | NM_006645 |
| A_23_P114883 | FMOD | NM_002023 | A_23_P399265 | STMN2 | S82024 |
| A_24_P347431 | FOXA1 | NM_004496 | A_23_P212500 | TF | NM_001063 |
| A_23_P384761 | GATA4 | NM_002052 | A_23_P101013 | TMC6 | NM_007267 |
| A_23_P129064 | GATM | NM_001482 | A_23_P409093 | TMEM16D | NM_178826 |
| A_23_P52227 | GDF10 | NM_004962 | A_32_P7015 | TSPAN15 | NM_012339 |
| A_23_P250444 | GJB1 | NM_000166 | A_23_P130333 | TTR | NM_000371 |
| A_32_P19294 | GLT1D1 | NM_144669 | A_23_P212968 | UGT2B11 | NM_001073 |
| A_32_P109029 | GPRC5C | NM_022036 | A_23_P16866 | VIL1 | NM_007127 |
| A_23_P253495 | GSTA3 | NM_000847 | A_23_P78099 | VTN | NM_000638 |

Table 11 below lists the genes expressed in induced human hepatic stem cells (CC1-4) that were derived from cancer tissues of a colon cancer patient and which were induced in Example 5.
Induced human hepatic stem cells derived from cancer tissues of a colon cancer patient: CC1-4
The number of expressed probes characteristic of hepatocytes was 92.

**[Table 11]**

| ProbeName | GeneSymbol | GenbankAccession | ProbeName | GeneSymbol | GenbankAccession |
|---|---|---|---|---|---|
| A_23_P116898 | A2M | NM_000014 | A_24_P52697 | H19 | NR_002196 |
| A_23_P252981 | ACE2 | NM_021804 | A_23_P47034 | HHEX | NM_002729 |
| A_24_P945113 | ACVRL1 | NM_000020 | A_23_P206760 | HP | NM_005143 |
| A_32_P196263 | ADAMTS9 | NM_182920 | A_23_P421493 | HPR | NM_020995 |
| A_23_P406341 | AFAP1L2 | NM_001001936 | A_23_P161998 | HPX | NM_000613 |
| A_23_P58205 | AFP | NM_001134 | A_23_P395438 | HTRA3 | NM_053044 |
| A_23_P115261 | AGT | NM_000029 | A_23_P150609 | IGF2 | NM_001007139 |
| A_23_P155514 | AHSG | NM_001622 | A_23_P15146 | IL32 | NM_001012631 |
| A_23_P155509 | AHSG | NM_001622 | A_23_P153964 | INHBB | NM_002193 |
| A_24_P766716 | AK126405 | AK126405 | A_23_P501193 | KCNJ16 | NM_170741 |
| A_23 P257834 | ALB | NM_000477 | A_23_P56898 | KYNU | NM_003937 |
| A_23_P83098 | ALDH1A1 | NM_000689 | A_23_P201636 | LAMC2 | NM_005562 |
| A_32_P105549 | ANXA8 | NM_001630 | A_23_P120902 | LGALS2 | NM_006498 |
| A_23_P337262 | APCDD1 | NM_153000 | A_24_P178834 | LOC132205 | AK091178 |
| A_23_P203191 | APOA1 | NM_000039 | A_24_P845223 | M27126 | M27126 |
| A_24_P302249 | APOA2 | NM_001643 | A_23_P164057 | MFAP4 | NM_002404 |
| A_23_P87036 | APOA4 | NM_000482 | A_23_P213171 | MTTP | NM_000253 |
| A_24_P753592 | BC018589 | BC018589 | A_23_P102364 | NGEF | NM_019850 |
| A_23_P143331 | BMP2 | NM 001200 | A_23_P360797 | NTF3 | NM_002527 |
| A_23_P75790 | C11orf9 | NM_013279 | A_24_P220485 | OLFML2A | NM_182487 |
| A_23_P88678 | C15orf27 | NM_152335 | A_23_P347070 | PAG1 | NM_018440 |
| A_23_P101407 | C3 | NM_000064 | A_23_P52121 | PDZK1 | NM_002614 |
| A_23_P71855 | C5 | NM_001735 | A_23_P146554 | PTGDS | NM_000954 |
| A_23_P33723 | CD163 | NM_004244 | A_23_P167030 | PTHR1 | NM_000316 |
| A_23_P217379 | COL4A6 | NM_033641 | A_23_P118392 | RASD1 | NM_016084 |
| A_23_P120125 | COLEC11 | NM_199235 | A_23_P75283 | RBP4 | NM_006744 |
| A_23_P213745 | CXCL14 | NM_004887 | A_23_P3934 | RNF43 | NM_017763 |
| A_23_P102000 | CXCR4 | NM_001008540 | A_23_P88849 | RRAD | NM_004165 |
| A_23_P131676 | CXCR7 | NM_020311 | A_23_P124619 | S100A14 | NM_020672 |
| A_23_P257583 | DENND2A | NM_015689 | A_23_P121926 | SEPP1 | NM_005410 |
| A_23_P105923 | DIO3 | NM_001362 | A_24_P145629 | SERINC2 | NM_178865 |
| A_24_P236251 | DLK1 | NM_003836 | A_23_P218111 | SERPINA1 | NM_001002236 |
| A_23_P139704 | DUSP6 | NM_001946 | A_23_P205355 | SERPINA5 | NM_000624 |
| A_23_P205177 | F10 | NM_000504 | A_23_P102391 | SLC40A1 | NM_014585 |
| A_23_P94879 | F2 | NM_000506 | A_24_P242581 | SLC5A9 | NM_001011547 |
| A_23_P79562 | FABP1 | NM_001443 | A_32_P133072 | SPON1 | NM_006108 |
| A_23_P166109 | FLRT3 | NM_198391 | A_23_P36345 | STARD10 | NM_006645 |
| A_23_P114883 | FMOD | NM_002023 | A_23_P399265 | STMN2 | S82024 |
| A_23_P37127 | FOXA1 | NM_004496 | A_23_P212500 | TF | NM_001063 |
| A_24_P347431 | FOXA1 | NM_004496 | A_23_P212508 | TF | NM_001063 |
| A 23_P91552 | FTCD | NM_206965 | A_23_P101013 | TMC6 | NM_007267 |
| A_23_P384761 | GATA4 | NM_002052 | A_23_P130333 | TTR | NM_000371 |
| A_23_P129064 | GATM | NM_001482 | A_23_P214408 | UNC93A | NM 018974 |
| A_32_P19294 | GLT1D1 | NM_144669 | A_24_P103434 | UNC93A | NM_018974 |
| A_32_P109029 | GPRC5C | NM_022036 | A_23_P16866 | VIL1 | NM_007127 |
| A_23_P69573 | GUCY1A3 | NM_000856 | A_23_P78099 | VTN | NM_000638 |

### 2. Genes characteristically expressed in human embryonic stem cells

All of the 31 expressed probes (23 genes: Table 12) on the Whole Human Genome Oligo DNA Microarray (4X44K) manufactured by Agilent Technologies, which were characteristically expressed in human embryonic stem cells, were expressed in the induced human hepatic stem cells of Examples 2-6 in almost comparable amounts (1/4-4 times) to those expressed in human embryonic stem cells. The Probe names, GeneSymbols, and GeneBank Accession Nos. for the genes in human embryonic stem cells are listed below.

**[Table 12]**

| ProbeName | GeneSymbol | GenbankAccession |
|---|---|---|
| A_24_P231132 | ACVR2B | NM_001106 |
| A_23_P109950 | ACVR2B | NM_001106 |
| A_32_P134209 | ACVR2B | NM_001106 |
| A_23_P85250 | CD24 | L33930 |
| A_23_P206359 | CDH1 | NM_004360 |
| A_23_P138655 | CYP26A1 | NM_057157 |
| A_23_P28953 | DNMT3B | NM_175850 |
| A_23_P380526 | DPPA4 | NM_018189 |
| A_23_P2831 | EDNRB | NM_003991 |
| A_24_P42755 | FLT1 | NM_002019 |
| A_23_P14821 | GABRB3 | NM_000814 |
| A_23_P10966 | GABRB3 | NM_000814 |
| A_23_P304450 | GATA6 | NM_005257 |
| A_23_P72817 | GDF3 | NM_020634 |
| A_23_P163992 | GRB7 | NM_005310 |
| A_23_P74895 | UN28 | NM_024674 |
| A_23_P204640 | NANOG | NM_024865 |
| A_23_P127322 | NODAL | NM_018055 |
| A_23_P215060 | PODXL | NM_005397 |
| A_24_P144601 | POU5F1 | NM_002701 |
| A_23_P59138 | POU5F1 | NM_002701 |
| A_32_P132563 | POU5F1 | NM_002701 |
| A_24_P214841 | POU5F1 | NM_002701 |
| A_23_P109072 | SALL4 | NM_020436 |
| A_23_P401055 | SOX2 | NM_003106 |
| A_24_P379969 | SOX2 | NM_003106 |
| A_32_P135985 | TDGF1 | NM_003212 |
| A_23_P366376 | TDGF1 | NM_003212 |
| A_23_P110851 | TERT | NM_198253 |
| A_23_P395582 | ZFP42 | NM_174900 |
| A_23_P327910 | ZIC3 | NM_003413 |

The above results experimentally verify that induced human hepatic stem cells not only gave rise to the expression of hepatocyte marker genes which is a property of hepatocytes, but also expressed genes characteristic of embryonic stem cells in comparable amounts to human embryonic stem cells.

Further analysis of the microarray results showed that the induced hepatic stem cell of the present invention has properties characteristic of mesendodermal stem cells and endodermal stem cells. More specifically, the induced hepatic stem cell expressed all of the SOX17 gene, the FOXA2 gene, the GSC gene, the EOMES gene, and the TCF2 gene which are genes characteristically expressed in mesendodermal stem cells and endodermal stem cells. Among the induced human hepatic stem cells (NFB1-3, GC1-2, NGC1-2, AFB1-1, and CC1-4) prepared in Examples 2-6, two cells (NFB1-3 and CC1-4) which harbored and therefore expressed relatively small amounts of genes characteristic of hepatocytes expressed the SOX17 gene, the FOXA2 gene, the GSC gene, the EOMES gene, and the TCF2 gene in greater amounts than other induced human hepatic stem cells (GC1-2, NGC1-2, and AFB1-1).

As described above, it was confirmed that induced human hepatic stem cells (NGC1-2) prepared from stomach cancer patient-derived non-cancer tissues and induced human hepatic stem cells (AFB1-1) prepared from adult skin tissues not only expressed alpha-fetoprotein (AFP), transthyretin (TTR), albumin (ALB), and alpha 1-antitrypsin (AAT) which are marker genes for hepatocytes, but also expressed the POU5F1 gene, the SOX2 gene, the NANOG gene, and the ZFP42 gene which are genes characteristic of embryonic stem cells in comparable amounts to human embryonic stem cells. It should be noted that AAT is sometimes designated as SERPINA1, transthyretin as prealbumin, and ZFP42 as REX1.

### EXAMPLE 9

### 9. Quantitative detection of hepatocyte markers and embryonic stem cell markers by immunofluorescent staining

Induced human hepatic stem cells (NGC1-2) prepared from stomach cancer patient-derived non-cancer tissues induced in Example 4, and induced human hepatic stem cells (AFB1-1) prepared from adult skin tissues induced in Example 6, were seeded onto the Lab-Tek (registered trademark) Chamber Slide (registered trademark) System (Nunc; Cat No. 177429). On the next day, after removing the medium from the cultured cells and washing the cells twice with PBS (-), a 10% formaldehyde solution was added, and the mixture was left to stand at room temperature for 15 minutes. Next, after removing the 10% formaldehyde solution , and washing three times with PBS (-), a 0.1 % Triton-X100 solution (ICN Biomedical) was added, and the mixture was left to stand at room temperature for 15 minutes. Then, after removing the 0.1% Triton-X100 solution , and washing three times with PBS (-), a blocking solution (in TBS; pH7.2) (Nacalai Tesque; Cat No. 05151-35) was added, and the mixture was left to stand at room temperature for one hour. Reaction with a 1:50 diluted primary antibody was performed at 4°C overnight or at room temperature for one hour. Then, after washing twice with PBS (-), reaction with a 1:500 diluted secondary antibody was performed at room temperature for 30 minutes. The primary and secondary antibodies used are as described below.

### <Primary antibodies>

Goat anti-human NANOG antibody (R&D Systems; Lot No. KKJ03), mouse anti-human SSEA-4 antibody (Millipore; Lot No. LV1488380), mouse anti-human CD9 antibody (R&D Systems; Lot No. JOK04), rabbit anti-human α-1-fetoprotein antibody (Dako; Lot No. A0008), mouse anti-human albumin antibody (Sigma-Aldrich; Lot No. A6684)

### <Secondary antibodies>

Donkey anti-goat IgG antibody labeled with Alexa Fluor 594 (Invitrogen; Cat No. A11058), goat anti-mouse IgG antibody labeled with Alexa Fluor 488 (Invitrogen; Cat No. A11001), goat anti-mouse IgG antibody labeled with Alexa Fluor 594 (Invitrogen; Cat No. A11005), donkey anti-rabbit IgG antibody labeled with Alexa Fluor 488 (Invitrogen; Cat No. A21206)

After staining, observation was made under a fluorescent microscope, and as a result, it was found that the induced human hepatic stem cells prepared from stomach cancer patient-derived non-cancer tissues and the induced human hepatic stem cells prepared from adult skin tissues exhibited a property of hepatocytes, namely the production of alpha-fetoprotein (AFP) and albumin (ALB) proteins, and expressed glycolipids, NANOG, SSEA-4, and CD9 which are characteristic of embryonic stem cells (not shown).

### EXAMPLE 10

### 10. Coexpression of the CD81 gene, the SCARB1 gene, the OCLN gene, and the CLDN1 genre

The CD81 gene, the SCARB1 gene, the OCLN gene, and the CLDN1 gene which are important genes for the replication of hepatitis C virus (HCV) were analyzed using the Whole Human Genome Oligo DNA Microarray (4X44K) manufactured by Agilent Technologies. The analysis software used was GeneSpring GX 10.0 (Agilent Technologies, Inc.), and normalization was performed using the 50th percentile method. The testing procedure was the same as that in Example 8.

### <Results of quantitative analysis for genes>

The microarray data for three human embryonic stem cells (i.e., hES_H9 (GSM194390), hES_BG03 (GSM194391), and hES_ES01 (GSM194392)) and induced pluripotent stem cells (i.e., iPS cells 201B7 (GSM241846)) to be used was downloaded from GEO.

It was confirmed and experimentally verified that induced human hepatic stem cells coexpressed the CD81 gene, the SCARB1 gene, the OCLN gene, and the CLDN1 gene which are important genes for the replication of hepatitis C virus (HCV). Accordingly, it was suggested that a test to evaluate the efficacy of an antiviral drug candidate compound can be conducted by infecting the induced hepatic stem cell of the present invention with hepatitis C virus and replicating the infected cell in the presence of the added compound. The same application was also suggested for human embryonic stem cells and induced human pluripotent stem cells.

In the induction of the induced hepatic stem cell of the present invention, it is necessary to bring the mammalian cell to such a state that the gene products of the POU5F1 gene, the KLF4 gene, and the SOX2 gene which are necessary for induction to the induced hepatic stem cell will be present to ensure that the intracellular relative abundance of the gene product of the POU5F1 gene is greater than that of the gene product of the SOX2 gene. In the present invention, the intracellular relative abundances of the gene products of the POU5F1 gene, the KLF4 gene, and the SOX2 gene preferably satisfies the relation of POU5F1 gene > KLF4 gene > SOX2 gene, and from the viewpoint of high-efficiency induction to the induced hepatic stem cell, the intracellular relative abundances of the gene products of the POU5F1 gene, the KLF4 gene, and the SOX2 gene most preferably assume values of 4, 2 and 1 in that order.

Further, in the present invention, for example, the NANOG gene, the POU5F1 gene, the SOX2 gene, the ZFP42 gene, the SALL4 gene, the LIN28 gene, and the TERT gene which are characteristically expressed in embryonic stem cells serve as "self-replication genes" which allow cells in various organisms to self-replicate *ex vivo.*

In the induction of the induced hepatic stem cell of the present invention, the intracellular relative abundances of the gene products of the POU5F1 gene, the KLF4 gene, and the SOX2 gene were held to be one of important factors in determining the ultimate course of differentiation, and in the preparation of the pluripotent stem cell, it was found that the intracellular relative abundances of the gene products of the POU5F1 gene, the KLF4 gene, and the SOX2 gene assumed values of 1, 1 and 1 in that order, and that the pluripotent stem cell was undifferentiated.

### INDUSTRIAL APPLICABILITY

According to the present invention, it is possible to prepare induced human hepatic stem cells from donors of different races, sexes, ages, or genetic backgrounds (such as polymorphisms), and therefore to evaluate and predict the efficacy, safety, toxicity and drug interaction of a candidate drug in a non-clinical test prior to evaluating these-features of the candidate drug administered to various patients in a clinical test. Accordingly, the induced human hepatic stem cell of the present invention is industrially very useful because they serve as a tool for drug discovery which contributes to improved efficiency in drug development and reduced burden on patients.

The induced hepatic stem cell of the present invention is very useful in search and analysis of molecules that control the formation and functions of the liver: for example, discovery of drugs for hepatic fibrosis, cirrhosis, fatty liver, hepatitis, metabolic syndrome, hematopoiesis and the like; analysis of the metabolism and mechanism of action of various pharmaceuticals and compounds; preparation of vaccines, and application to bioreactors.

## Claims

1. An induced hepatic stem cell obtained by a process comprising a step of inducing a mammalian cell into an induced hepatic stem cell,
wherein the mammalian cell is selected from the group consisting of an adult-derived cell, a neonate-derived cell, a skin-derived cell, and a cell from a cancerous individual,
wherein said step of inducing comprises introducing a POU5F1 gene, a KLF4 gene, and a SOX2 gene into the mammalian cell with the aid of expression vectors to bring the mammalian cell to such a state that gene products of the POU5F1 gene, the KLF4 gene, and the SOX2 gene which are necessary for induction to the induced hepatic stem cell will be present to ensure that the intracellular relative abundance of the gene product of the POU5F1 gene is greater than that of the gene product of SOX2 gene,
wherein the ratio in use between the POU5F1 gene, the KLF4 gene, and the SOX2 gene which are necessary for induction to the induced hepatic stem cell is 4:2:1 in that order, and
wherein the induced hepatic stem cell is **characterized by** at least satisfying the following requirements (1)-(3):
(1) it expresses at least 15 genes as selected from the group of the genes listed in the following Table 1 which are marker genes for an embryonic stem cell;
**[Table 1]**
| GeneSymbol | GenbankAccession |
|---|---|
| ACVR2B | NM_001106 |
| CD24 | L33930 |
| CDH1 | NM_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | NM_020634 |
| GRB7 | NM_005310 |
| UN28 | NM_024674 |
| NANOG | NM_024865 |
| NODAL | NM_018055 |
| PODXL | NM_005397 |
| POU5F1 | NM_002701 |
| SALL4 | NM_020436 |
| SOX2 | NM_003106 |
| TDGF1 | NM_003212 |
| TERT | NM_198253 |
| ZFP42 | NM_174900 |
| ZIC3 | NM_003413 |
(2) it has properties of a hepatocyte wherein at least 15 genes as selected from the gene group in Table 2 below are expressed as genes associated with the properties of a hepatocyte;
**[Table 2]**
| GeneSymbol | GenbankAccession | GeneSymbol | GenbankAccession | GeneSymbol | GenbankAccession |
|---|---|---|---|---|---|
| A2M | NM_000014 | ERP27 | NM_152321 | NRCAM | NM_005010 |
| ACE2 | NM_021804 | EVA1 | NM_144765 | NTF3 | NM_002527 |
| ACVRL1 | NM_000020 | F10 | NM_000504 | OLFML2A | NM_182487 |
| ADAMTS9 | NM_182920 | F2 | NM_000506 | PAG1 | NM_018440 |
| AFAP1L2 | NM_001001936 | FABP1 | NM_001443 | PCSK6 | NM_002570 |
| AFP | NM_001134 | FGA | NM_021871 | PDK4 | NM_002612 |
| AGT | NM_000029 | FGA | NM_000508 | PDZK1 | NM_002614 |
| AHSG | NM_001622 | FGB | NM_005141 | PLA2G12B | NM_032562 |
| AK027294 | AK027294 | FGG | NM_000509 | PLG | NM_000301 |
| AK074614 | AK074614 | FLRT3 | NM_198391 | PRG4 | NM_005807 |
| AK124281 | AK124281 | FMOD | NM_002023 | PSMAL | NM_153696 |
| AK126405 | AK126405 | FOXA1 | NM_004496 | PTGDS | NM_000954 |
| ALB | NM_000477 | FTCD | NM_206965 | PTHR1 | NM_000316 |
| ALDH1A1 | NM_000689 | GATE4 | NM_002052 | RASD1 | NM_016084 |
| ANXA8 | NM_001630 | GATM | NM_001482 | RBP4 | NM_006744 |
| APCDD1 | NM_153000 | GDF10 | NM_004962 | RNF43 | NM_017763 |
| APOA1 | NM_000039 | GJB1 | NM_000166 | RRAD | NM_004165 |
| APOA2 | NM_001643 | GLT1D1 | NM_144669 | S100A14 | NM_020672 |
| APOA4 | NM_000482 | GPRC5C | NM_022036 | SEPP1 | NM_005410 |
| APOB | NM_000384 | GSTA3 | NM_000847 | SERINC2 | NM_178865 |
| AREG | NM_001657 | GUCY1A3 | NM_000856 | SERPINA1 | NM_001002236 |
| ART4 | NM_021071 | H19 | NR_002196 | SERPINA3 | NM_001085 |
| ASGR2 | NM_080912 | HHEX | NM_002729 | SERPINA5 | NM_000624 |
| ATAD4 | NM_024320 | HKDC1 | NM_025130 | SH3TC1 | NM_018986 |
| BC018589 | BC018589 | HMGCS2 | NM_005518 | SLC13A5 | NM_177550 |
| BMP2 | NM_001200 | HP | NM_005143 | SLC40A1 | NM_014585 |
| BX097190 | BX097190 | HPR | NM_020995 | SLC5A9 | NM_001011547 |
| C11orf9 | NM_013279 | HPX | NM_000613 | SLCO2B1 | NM_007256 |
| C13orf15 | NM_014059 | HSD17B2 | NM_002153 | SLPI | NM_003064 |
| C15orf27 | NM_152335 | HTRA3 | NM_053044 | SPARCL1 | NM_004684 |
| C3 | NM_000064 | IGF2 | NM_001007139 | SPON1 | NM_006108 |
| C5 | NM_001735 | IL32 | NM_001012631 | ST8SIA1 | NM_003034 |
| CA414006 | CA414006 | INHBB | NM_002193 | STARD10 | NM_006645 |
| CD163 | NM_004244 | ISX | NM_001008494 | STMN2 | S82024 |
| CD1D | NM_001766 | KCNJ16 | NM_170741 | TD02 | NM_005651 |
| CDX2 | NM_001265 | KYNU | NM_003937 | TF | NM_001063 |
| CILP | NM_003613 | LAMC2 | NM_005562 | TMC6 | NM_007267 |
| CMKLR1 | NM_004072 | LGALS2 | NM_006498 | TMEM16D | NM_178826 |
| COL4A6 | NM_033641 | LHX2 | NM_004789 | TSPAN15 | NM_012339 |
| COLEC11 | NM_199235 | LOC132205 | AK091178 | TTR | NM_000371 |
| CXCL14 | NM_004887 | LOC285733 | AK091900 | UBD | NM_006398 |
| CXCR4 | NM_001008540 | M27126 | M27126 | UGT2B11 | NM_001073 |
| CXCR7 | NM_020311 | MAF | AF055376 | UGT2B7 | NM_001074 |
| DACH1 | NM_080759 | MFAP4 | NM_002404 | UNC93A | NM_018974 |
| DENND2A | NM_015689 | MMP10 | NM_002425 | VCAM1 | NM_001078 |
| DIO3 | NM_001362 | MTTP | NM_000253 | VIL1 | NM_007127 |
| DLK1 | NM_003836 | NGEF | NM_019850 | VTN | NM_000638 |
| DUSP6 | NM_001946 | NGFR | NM_002507 | WFDC1 | NM_021197 |
(3) it can be subjected to expansion culture or passage culture for at least 3 days.

2. The induced hepatic stem cell as recited in claim 1, wherein the marker genes for an embryonic stem cell in (1) above are expressed in the induced hepatic stem cell in amounts ranging from 1/8-8 times the amounts of the genes that are expressed in the embryonic stem cell.

3. The induced hepatic stem cell as recited in claim 2, wherein the marker genes for an embryonic stem cell in (1) above are expressed in the induced hepatic stem cell in amounts ranging from 1/4-4 times the amounts of the genes that are expressed in the embryonic stem cell.

4. The induced hepatic stem cell as recited in any one of claims 1 to 3, wherein the NANOG gene, POU5F1 gene, SOX2 gene, ZFP42 gene, and SALL4 gene are expressed as the marker genes for an embryonic stem cell in (1) above.

5. The induced hepatic stem cell as recited in any one of claims 1 to 4, wherein the AFP gene, TTR gene, TF gene, APOA2 gene, APOA4 gene, AHSG gene, FGA gene, AGT gene, FABP1 gene, SERPINA1 gene, and RBP4 gene are expressed as genes associated with the properties of a hepatocyte in (2) above.

6. The induced hepatic stem cell as recited in any one of claims 1 to 4, wherein the APOA1 gene, APOA2 gene, APOA4 gene, APOB gene, FABP1 gene, and AGT gene are expressed as genes associated with the properties of a hepatocyte in (2) above.

7. The induced hepatic stem cell as recited in any one of claims 1 to 6, which further expresses at least one gene as selected from among the SOX17 gene, FOXA2 gene, GSC gene, EOMES gene, and TCF2 gene which are characteristic of mesendodermal stem cells and/or endodermal stem cells.

8. The induced hepatic stem cell as recited in claim 1, wherein the mammalian cell is a human cell.

9. An in vitro method using the induced hepatic stem cell as recited in claim 1, which is selected from among a safety test method, a toxicity test method, a metabolism test method, a drug interaction test method, an antiviral activity test method, and a screening test method for pharmaceuticals.

10. The induced hepatic stem cell as recited in claim 1 for use in a therapeutic method of treatment of a mammal, wherein the induced hepatic stem cell is to be transplanted into the liver of the mammal.

## Patentansprüche

1. Induzierte hepatische Stammzelle, erhalten durch ein Verfahren, das den Schritt des Induzierens einer Säugerzelle in eine induzierte hepatische Stammzelle umfasst,
wobei die Säugerzelle ausgewählt ist aus der Gruppe, bestehend aus einer von einem Erwachsenen stammenden Zelle, einer von einem Neugeborenen stammenden Zelle, einer von der Haut stammenden Zelle und einer Zelle eines Krebspatienten,
wobei der Schritt des Induzierens das Einbringen eines POU5F1 Gens, eines KLF4 Gens und eines SOX2 Gens in die Säugerzelle mit der Hilfe von Expressionsvektoren umfasst, um die Säugerzelle in einen Zustand zu versetzen, in dem die Genprodukte des POU5F1 Gens, des KLF4 Gens und des SOX2 Gens, die für die Induktion in die induzierte hepatische Stammzelle notwendig sind, anwesend sein werden, so dass sicher gestellt ist, dass die intrazelluläre relative Menge des Genprodukts des POU5F1 Gens größer als dasjenige Genprodukt des SOX2 Gens ist, wobei das verwendete Verhältnis zwischen dem POU5F1 Gen, dem KLF4 Gen und dem SOX2 Gen, die für die Induktion in die induzierte hepatische Stammzelle notwendig sind, in dieser Reihenfolge 4:2:1 ist, und
wobei die induzierte hepatische Stammzelle **dadurch gekennzeichnet ist, dass** sie zumindest die folgenden Voraussetzungen (1)-(3) erfüllt;
(1) sie exprimiert mindestens 15 Gene, die ausgewählt sind aus der Gruppe von Genen in der folgenden Tabelle 1, die Markergene embryonaler Stammzellen sind;
**[Tabelle 1]**
| Gen-Symbol | Genbank-Zugang |
|---|---|
| ACVR2B | NM_001106 |
| CD24 | L33930 |
| CDH1 | NM_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | MM_020634 |
| GRB7 | NM_005310 |
| LIN28 | NM_024674 |
| NANOG | NM_024865 |
| NODAL | NM_018055 |
| PODXL | NM_005397 |
| POU5F1 | NM_002701 |
| SALL4 | NM_020436 |
| SOX2 | NM_003106 |
| TDGF1 | NM_003212 |
| TERT | NM_198253 |
| ZFP42 | NM_174900 |
| ZIC3 | NM_003413 |
(2) sie hat Eigenschaften eines Hepatozyten, wobei mindestens 15 Gene, ausgewählt aus der Gengruppe in der nachstehenden Tabelle 2 als Gene, die mit den Eigenschaften von einem Hepatozyten assoziiert sind, exprimiert werden;
**[Tabelle 2]**
| Gen-Symbol | Genbank-Zugang | Gen-Symbol | Genbank-Zugang | Gen-Symbol | Genbank-Zugang |
|---|---|---|---|---|---|
| A2M | NM_000014 | ERP27 | NM_152321 | NRCAM | NM_005010 |
| ACE2 | NM_021804 | EVA1 | NM_144765 | NTF3 | NM_002527 |
| ACVRL1 | NM_000020 | F10 | NM_000504 | OLFML2A | NM_182487 |
| ADAMTS9 | NM_182920 | F2 | NM_000506 | PAG1 | NM_018440 |
| AFAP1L2 | NM_001001936 | FABP1 | NM_001443 | PCSK6 | NM_002570 |
| AFP | NM_001134 | FGA | NM_021871 | PDK4 | NM_002612 |
| AGT | NM_000029 | FGA | NM_000508 | PDZK1 | NM_002614 |
| AHSG | NM_001622 | FGB | NM_005141 | PLA2G12B | NM_032562 |
| AK027294 | AK027294 | FGG | NM_000509 | PLG | NM_000301 |
| AK074614 | AK074614 | FLRT3 | NM_198391 | PRG4 | NM_005807 |
| AK124281 | AK124281 | FMOD | NM_002023 | PSMAL | NM_153696 |
| AK126405 | AK126405 | FOXA1 | NM_004496 | PTGDS | NM_000954 |
| ALB | NM_000477 | FTCD | NM_206965 | PTHR1 | NM_000316 |
| ALDH1A1 | NM_000689 | GATA4 | NM_002052 | RASD1 | NM_016084 |
| ANXA8 | NM_001630 | GATM | NM_001482 | RBP4 | NM_006744 |
| APCDD1 | NM_153000 | GDF10 | NM_004962 | RNF43 | NM_017763 |
| APOA1 | NM_000039 | GJB1 | NM_000166 | RRAD | NM_004165 |
| APOA2 | NM_001643 | GLT1D1 | NM_144669 | S100A14 | NM_020672 |
| APOA4 | NM_000482 | GPRC5C | NM_022036 | SEPP1 | NM_005410 |
| APOB | NM_000384 | GSTA3 | NM_000847 | SERINC2 | NM_178865 |
| AREG | NM_001657 | GUCY1A3 | NM_000856 | SERPINA1 | NM_001002236 |
| ART4 | NM_021071 | H19 | NR_002196 | SERPINA3 | NM_001085 |
| ASGR2 | NM_080912 | HHEX | NM_002729 | SERPINA5 | NM_000624 |
| ATAD4 | NM_024320 | HKDC1 | NM_025130 | SH3TC1 | NM_018986 |
| BC018589 | BC018589 | HMGCS2 | NM_005518 | SLC13A5 | NM_177550 |
| BMP2 | NM_001200 | HP | NM_005143 | SLC40A1 | NM_014585 |
| BX097190 | BX097190 | HPR | NM_020995 | SLC5A9 | NM_001011547 |
| C11orf9 | NM_013279 | HPX | NM_000613 | SLCO2B1 | NM_007256 |
| C13orf15 | NM_014059 | HSD17B2 | NM_002153 | SLPI | NM_003064 |
| C15orf27 | NM_152335 | HTRA3 | NM_053044 | SPARCL1 | NM_004684 |
| C3 | NM_000064 | IGF2 | NM_001007139 | SPON1 | NM_006108 |
| C5 | NM_001735 | IL32 | NM_001012631 | ST8SIA1 | NM_003034 |
| CA414006 | CA414006 | INHBB | NM_002193 | STARD10 | NM_006645 |
| CD163 | NM_004244 | ISX | NM_001008494 | STMN2 | S82024 |
| CD1D | NM_001766 | KCNJ16 | NM_170741 | TDO2 | NM_005651 |
| CDX2 | NM_001265 | KYNU | NM_003937 | TF | NM_001063 |
| CILP | NM_003613 | LAMC2 | NM_005562 | TMC6 | NM_007267 |
| CMKLR1 | NM_004072 | LGALS2 | NM_006498 | TMEM16D | NM_178826 |
| COL4A6 | NM_033641 | LHX2 | NM_004789 | TSPAN15 | NM_012339 |
| COLEC11 | NM_199235 | LOC132205 | AK091178 | TTR | NM_000371 |
| CXCL14 | NM_004887 | LOC285733 | AK091900 | UBD | NM_006398 |
| CXCR4 | NM_001008540 | M27126 | M27126 | AGT2811 | NM_001073 |
| CXCR7 | NM_020311 | MAF | AF055376 | UGT2B7 | NM_001074 |
| DACH1 | NM_080759 | MFAP4 | NM_002404 | UNC93A | NM_018974 |
| DENND2A | NM_015689 | MMP10 | NM_002425 | VCAM1 | NM_001078 |
| DIO3 | NM_001362 | MTTP | NM_000253 | VIL1 | NM_007127 |
| DLK1 | NM_003836 | NGEF | NM_019850 | VTN | NM_000638 |
| DUSP6 | NM_001946 | NGFR | NM_002507 | WFDC1 | NM_021197 |
(3) sie kann einer Expansionszüchtung oder einer Passagenzüchtung für mindestens 3 Tage ausgesetzt werden.

2. Induzierte hepatische Stammzelle nach Anspruch 1, wobei die Markergene für eine embryonale Stammzelle in (1), oben, in der induzierten hepatischen Stammzelle in Mengen exprimiert werden, die von 1/8-8 mal der Mengen der Gene reichen, die in der embryonalen Stammzelle exprimiert werden.

3. Induzierte hepatische Stammzelle nach Anspruch 2, wobei die Markergene für eine embryonale Stammzelle in (1), oben, in der induzierten hepatischen Stammzelle in Mengen exprimiert werden, die von 1/4-4 mal der Mengen der Gene reichen, die in der embryonalen Stammzelle exprimiert werden.

4. Induzierte hepatische Stammzelle nach einem der Ansprüche 1 bis 3, wobei das NANOG Gen, POU5F1 Gen, SOX2 Gen, ZFP42 Gen und SALL4 Gen als die Markergene für eine embryonale Stammzelle in (1), oben, exprimiert sind.

5. Induzierte hepatische Stammzelle nach einem der Ansprüche 1 bis 4, wobei das AFP Gen, TTR Gen, TF Gen, APOA2 Gen, APOA4 Gen, AHSG Gen, FGA Gen, AGT Gen, FABP1 Gen, SERPINA1 Gen und RBP4 Gen als Gene, die mit den Eigenschaften von Hepatozyten in (2), oben, assoziiert sind, in (2), oben, exprimiert sind.

6. Induzierte hepatische Stammzelle nach einem der Ansprüche 1 bis 4, wobei das APOA1 Gen, APOA2 Gen, APOA4 Gen, APOB Gen, FABP1 Gen und AGT Gen als Gene, die mit den Eigenschaften von Hepatozyten in (2), oben, assoziiert sind, in (2), oben, exprimiert sind.

7. Induzierte hepatische Stammzelle nach einem der Ansprüche 1 bis 6, die weiterhin mindestens ein Gen ausgewählt aus dem SOX17 Gen, FOXA2 Gen, GSC Gen, EOMES Gen, und TCF2 Gen exprimiert, die charakteristisch für mesodermale Stammzellen und/oder endodermale Stammzellen sind.

8. Induzierte hepatische Stammzelle nach Anspruch 1, wobei die Säugerzelle eine menschliche Zelle ist.

9. *In vitro* Verfahren, das die induzierte hepatische Stammzelle nach Anspruch 1 verwendet, das ausgewählt ist aus einem Sicherheitstestverfahren, einem Toxizitätstestverfahren, einem Metabolismustestverfahren, einem Medikamenten-Interaktionstestverfahren, einem antiviralen Aktivitätstestverfahren und einem Screeningtestverfahren für Arzneimittel.

10. Induzierte hepatische Stammzelle nach Anspruch 1 zur Verwendung in einem therapeutischen Verfahren zur Behandlung eines Säugers, in dem die induzierte hepatische Stammzelle in die Leber des Säugers zu implantieren ist.

## Revendications

1. Cellule souche hépatique induite obtenue par un procédé comprenant une étape d'induction d'une cellule de mammifère en une cellule souche hépatique induite, où la cellule de mammifère est choisie dans le groupe constitué d'une cellule dérivée d'un adulte, d'une cellule dérivée d'un nouveau-né, d'une cellule dérivée de la peau, et d'une cellule provenant d'un individu cancéreux,
où ladite étape d'induction comprend l'introduction d'un gène POU5F1, d'un gène KLF4, et d'un gène SOX2 dans la cellule de mammifère avec l'aide de vecteurs d'expression pour amener la cellule de mammifère à un état tel que les produits géniques du gène POU5F1, du gène KLF4, et du gène SOX2 qui sont nécessaires pour l'induction en la cellule souche hépatique induite seront présents pour assurer que l'abondance intracellulaire relative du produit génique du gène POU5F1 est supérieure à celle du produit génique du gène SOX2,
où le rapport dans l'utilisation entre le gène POU5F1, le gène KLF4, et le gène SOX2 qui sont nécessaires pour l'induction en la cellule souche hépatique induite est de 4/2/1 dans cet ordre, et
où la cellule souche hépatique induite est **caractérisée par** au moins la satisfaction des exigences (1) à (3) suivantes :
(1) elle exprime au moins 15 gènes tels que choisis dans le groupe des gènes énumérés dans le tableau 1 suivant qui sont des gènes marqueurs pour une cellule souche embryonnaire ;
**Tableau 1**
| Symbole du gène | Accession Genbank |
|---|---|
| ACVR2B | NM_001106 |
| CD24 | L33930 |
| CDH1 | NM_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | NM_020634 |
| GRB7 | NM_005310 |
| LIN28 | NM_024674 |
| NANOG | NM_024865 |
| NODAL | NM_018055 |
| PODXL | NM_005397 |
| POU5F1 | NM_002701 |
| SALL4 | NM_020436 |
| SOX2 | NM_003106 |
| TDGF1 | NM_003212 |
| TERT | NM_198253 |
| ZFP42 | NM_174900 |
| ZIC3 | NM_003413 |
(2) elle a des propriétés d'un hépatocyte dans lequel au moins 15 gènes tels que choisis dans le groupe des gènes dans le tableau 2 ci-dessous sont exprimés en tant que gènes associés aux propriétés d'un hépatocyte.
**Tableau 2**
| Symbole du gène | Accession Genbank | Symbole du gène | Accession Genbank | Symbole du gène | Accession Genbank |
|---|---|---|---|---|---|
| A2M | NM_000014 | ERP27 | NM_152321 | NRCAM | NM_005010 |
| ACE2 | NM_021804 | EVA1 | NM_144765 | NTF3 | NM_002527 |
| ACVRL1 | NM_000020 | F10 | NM_000504 | OLFML2A | NM_182487 |
| ADAMTS9 | NM_182920 | F2 | NM_000506 | PAG1 | NM_018440 |
| AFAP1L2 | NM_001001936 | FABP1 | NM_001443 | PCSK6 | NM_002570 |
| AFP | NM_001134 | FGA | NM_021871 | PDK4 | NM_002612 |
| AGT | NM_000029 | FGA | NM_000508 | PDZK1 | NM_002614 |
| AHSG | NM_001622 | FGB | NM_005141 | PLA2G12B | NM_032562 |
| AK027294 | AK027294 | FGG | NM_000509 | PLG | NM_000301 |
| AK074614 | AK074614 | FLRT3 | NM_198391 | PRG4 | NM_005807 |
| AK124281 | AK124281 | FMOD | NM_002023 | PSMAL | MM_153696 |
| AK126405 | AK126405 | FOXA1 | NM_004496 | PTGDS | NM_000954 |
| ALB | NM_000477 | FTCD | NM_206965 | PTHR1 | NM_000316 |
| ALDH1A1 | NM_000689 | GATA4 | NM_002052 | RASD1 | NM_016084 |
| ANXA8 | NM_001630 | GATM | NM_001482 | RBP4 | NM_006744 |
| APCDD1 | NM_153000 | GDF10 | NM_004962 | RNF43 | NM_017763 |
| APOA1 | NM_000039 | GJB1 | NM_000166 | RRAD | NM_004165 |
| APOA2 | NM_001643 | GLT1D1 | NM_144669 | S100A14 | NM_020672 |
| APOA4 | NM_000482 | GPRC5C | NM_022036 | SEPP1 | NM_005410 |
| APOB | NM_000384 | GSTA3 | NM_000847 | SERINC2 | NM_178865 |
| AREG | NM_001857 | GUCY1A3 | NM_000856 | SERPINA1 | NM_001002236 |
| ART4 | NM_021071 | H19 | NR_002196 | SERPINA3 | NM_001085 |
| ASGR2 | NM_080812 | HHEX | NM_002729 | SERPINA5 | NM_000624 |
| ATAD4 | NM_024320 | HKDC1 | MM_025130 | SH3TC1 | NM_018986 |
| BC018589 | BC018589 | HMGCS2 | NM_005518 | SLC13A5 | NM_177550 |
| BMP2 | NM_001200 | HP | NM_005143 | SLC40A1 | NM_014585 |
| BX097190 | BX097180 | HPR | NM_020995 | SLC5A9 | NM_001011547 |
| C11orf9 | NM_013278 | HPX | NM_000613 | SLCO2B1 | NM_007256 |
| C13orf15 | NM_014059 | HSD17B2 | NM_002153 | SLPI | NM_003064 |
| C15orf27 | NM_152335 | HTRA3 | NM_053044 | SPARCL1 | NM_004684 |
| C3 | NM_000064 | IGF2 | NM_001007138 | SPON1 | NM_006108 |
| C5 | NM_001735 | IL32 | NM_001012631 | ST8SIA1 | NM_003034 |
| CA414006 | CA414006 | INHBB | NM_002193 | STARD10 | NM_006645 |
| CD163 | NM_004244 | ISX | NM_001008494 | STMN2 | S82024 |
| CD1D | NM_001766 | KCNJ16 | NM_170741 | TDO2 | NM_005651 |
| CDX2 | NM_001265 | KYNU | NM_003937 | TF | NM_001063 |
| CILP | NM_003613 | LAMC2 | NM_005562 | TMC6 | NM_007267 |
| CMKLR1 | NM_004072 | LGALS2 | NM_006498 | TMEM16D | NM_178826 |
| COL4A6 | NM_033641 | LHX2 | NM_004789 | TSPAN15 | MM_012339 |
| COLEC11 | NM_199235 | LOC132205 | AK091178 | TTR | NM_000371 |
| CXCL14 | NM_004887 | LOC285733 | AK091900 | UBD | NM_006398 |
| CXCR4 | NM_001008540 | M27126 | M27126 | UGT2B11 | NM_001073 |
| CXCR7 | NM_020311 | MAF | AF055376 | UGT2B7 | NM_001074 |
| DACH1 | NM_080759 | MFAP4 | NM_002404 | UNC93A | NM_018974 |
| DENND2A | NM_015689 | MMP10 | NM_002425 | VCAM1 | NM_001078 |
| DIO3 | NM_001362 | MTTP | NM_000253 | VIL1 | NM_007127 |
| DLK1 | NM_003836 | NGEF | NM_019850 | VTN | NM_000638 |
| DUSP6 | NM_001946 | NGFR | NM_002507 | WFDC1 | NM_021197 |
(3) elle peut être soumise à une culture d'expansion ou une culture de passage pendant au moins 3 jours.

2. Cellule souche hépatique induite selon la revendication 1, dans laquelle les gènes marqueurs pour une cellule souche embryonnaire en (1) ci-dessus sont exprimés dans la cellule souche hépatique induite dans des quantités situées dans la plage de 1/8 à 8 fois les quantités des gènes qui sont exprimés dans la cellule souche embryonnaire.

3. Cellule souche hépatique induite selon la revendication 2, dans laquelle les gènes marqueurs pour une cellule souche embryonnaire en (1) ci-dessus sont exprimés dans la cellule souche hépatique induite dans des quantités situées dans la plage de 1/4 à 4 fois les quantités des gènes qui sont exprimés dans la cellule souche embryonnaire.

4. Cellule souche hépatique induite selon l'une quelconque des revendications 1 à 3, dans laquelle le gène NANOG, le gène POU5F1, le gène SOX2, le gène ZFP42, et le gène SALL4 sont exprimés en tant que gènes marqueurs pour une cellule souche embryonnaire en (1) ci-dessus.

5. Cellule souche hépatique induite selon l'une quelconque des revendications 1 à 4, dans laquelle le gène AFP, le gène TTR, le gène TF, le gène APOA2, le gène APOA4, le gène AHSG, le gène FGA, le gène AGT, le gène FABP1, le gène SERPINA1, et le gène RBP4 sont exprimés en tant que gènes associés aux propriétés d'un hépatocyte en (2) ci-dessus.

6. Cellule souche hépatique induite selon l'une quelconque des revendications 1 à 4, dans laquelle le gène APOA1, le gène APOA2, le gène APOA4, le gène APOB, le gène FABP1 et le gène AGT sont exprimés en tant que gènes associés aux propriétés d'un hépatocyte en (2) ci-dessus.

7. Cellule souche hépatique induite selon l'une quelconque des revendications 1 à 6, qui exprime en outre au moins un gène tel que choisi parmi le gène SOX17, le gène FOXA2, le gène GSC, le gène EOMES, et le gène TCF2 qui sont caractéristiques des cellules souches mésendodermiques et/ou des cellules souches endodermiques.

8. Cellule souche hépatique induite selon la revendication 1, où la cellule de mammifère est une cellule humaine.

9. Procédé *in vitro* utilisant la cellule souche hépatique induite selon la revendication 1, qui est choisi parmi un procédé de test d'innocuité, un procédé de test de toxicité, un procédé de test de métabolisme, un procédé de test d'interaction médicamenteuse, un procédé de test d'activité antivirale, et un procédé de test de criblage pour des agents pharmaceutiques.

10. Cellule souche hépatique induite selon la revendication 1, destinée à une utilisation dans un procédé thérapeutique de traitement d'un mammifère, où la cellule souche hépatique induite doit être transplantée dans le foie du mammifère.
